(19) Europäisches Patentamt
European Patent Office
Office européen des brevets

(11) Publication number: 0 013 067
A1

(12) # EUROPEAN PATENT APPLICATION

(21) Application number: 79302384.7

(22) Date of filing: 31.10.79

(51) Int. Cl.³: C 07 D 499/00
A 61 K 31/43
//C07D205/08, C07F7/18,
C07F9/65

(30) Priority: 22.12.78 GB 4984278

(43) Date of publication of application:
09.07.80 Bulletin 80/14

(84) Designated Contracting States:
BE CH DE FR GB IT NL SE

(71) Applicant: BEECHAM GROUP LIMITED
Beecham House Great West Road
Brentford, Middlesex(GB)

(72) Inventor: Perryman, Broom Nigel John
Martindale, Horsham Road
South Holmwood Dorking, Surrey(GB)

(74) Representative: Cole, William Gwyn et al,
Beecham Pharmaceuticals Yew Tree Bottom Road
Epsom Surrey KT18 5XQ(GB)

(54) **Bicyclic beta-lactam antibacterial agents, their use in pharmaceutical compositions, processes for their preparation and intermediates for use in such processes.**

(57) The compounds of the formula (II):

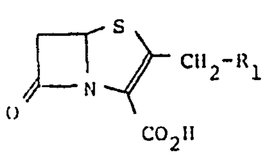

(II)

and salts and cleavable esters thereof wherein $R_1$ is a OH, $OR_2$, $OCOR_3$, $O.CO.OR_4$, $O.SO_2R_5$, $N_3$ or $NH_2$ group wherein $R_2$ is a lower alkyl group, a substituted lower alkyl group or an aralkyl group; $R_3$ is a lower alkyl group, a substituted lower alkyl group, an aryl group or an aralkyl group; $R_4$ is a lower alkyl group, a substituted lower alkyl group, an aryl group or an aralkyl group; and $R_5$ is a lower alkyl group, or a tolyl group are antibacterially effective agents. Process for their preparation and their use are described.

EP 0 013 067 A1

Bicyclic β-Lactam Antibacterial Agents, Their Use in Pharmaceutical Compositions, Processes for their Preparation and Intermediates for Use in Such Processes

R. B. Woodward (Acta Pharm. Suecica 1977, 14 Suppl., p 23 - 25) disclosed that the compounds of the formula (I):

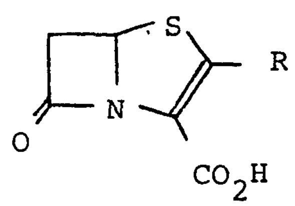

(I)

where R was an unspecified group possessed antibacterial activity. No aid in determining the nature of the group R was given by Professor Woodward nor did he describe the preparation of any compounds of the formula (I). However, at the symposium on Current Topics in Drug Research (Uppsala, Sweden October 1977) Professor Woodward described the compound of the formula (I) wherein R is a hydrogen atom. We have prepared this compound and found it to possess a somewhat disappointing degree of antibacterial activity. Clearly it would be desirable to find a class of compounds that possess a better degree of activity especially against Gram-

positive organisms such as Staphylococcus aureus, Streptococcus pneumoniae or Streptococcus pyogenes. Such a class of compounds has now been found.

Accordingly the present invention provides the compounds of the formula (II):

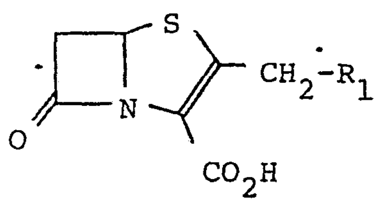

(II)

and salts and cleavable esters thereof wherein $R_1$ is a OH, $OR_2$, $OCOR_3$, $O.CO.OR_4$, $O.SO_2R_5$, $N_3$ or $NH_2$ group wherein $R_2$ is a lower alkyl group, a substituted lower alkyl group or an aralkyl group; $R_3$ is a lower alkyl group, a substituted lower alkyl group, an aryl group or an aralkyl group; $R_4$ is a lower alkyl group, a substituted lower alkyl group, an aryl group or an aralkyl group; and $R_5$ is a lower alkyl group, or a tolyl group.

When used herein the term "lower alkyl" means an alkyl group of 1 - 4 carbon atoms such as the methyl and ethyl groups, When used herein the term "aryl" means a phenyl group or a phenyl group substituted by a halogen atom or an alkoxyl group of 1 or 2 carbon atoms. When used herein the term "lower aralkyl" means a lower alkyl group substituted by an aryl group. When used herein the term "substituted lower alkyl" means a

lower alkyl group substituted by a methoxyl, ethoxyl, acetoxyl, amino, acetamido, benzyloxycarbonylamino, benzyloxycarbonyloxy or hydroxyl group of which the amino and hydroxyl groups must not be attached to the α-carbon atom.

Very favourably $R_1$ is a hydroxyl group as the resulting compound of the formula (III):

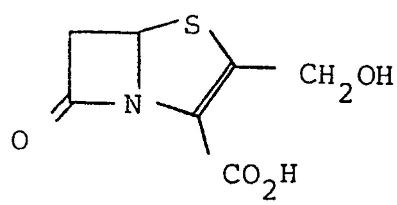

(III)

and its salts and cleavable esters serve as intermediates as well as biologically active substances.

Preferably $R_1$ is amino as the resulting compounds have particularly good antibacterial properities, for example against Pseudomonas.

Suitable values for $R_2$ include the methyl, ethyl, benzyl, 2-hydroxyethyl, 2-aminoethyl, 2-acetoxymethyl 2-methoxyethyl, acetoxymethyl, methoxymethyl,-and methane sulphonyl groups.

Suitable values for $R_3$ and $R_4$ include those listed as suitable for $R_2$ and the phenyl group.

Suitable values for $R_5$ include the methyl and p-tolyl groups. Such compounds are intended primarily as intermediates, for example in the preparation of the compounds in which $R_1$ is $N_3$. The azide is also envisaged primarily as an intermediate in the preparation of the corresponding compound in which $R_1$ is $NH_2$.

It is believed that the compounds of the formula (II) or their salts are the antibacterially active species and that biologically cleavable esters thereof act as pro-drugs. Thus in one favoured aspect this invention provides the compounds of the formula (II)

wherein $R_1$ is as hereinbefore defined and pharmaceutically acceptable salts thereof. Non-pharmaceutically acceptable salts of the compounds of the formula (II) also form part of this invention since they may be used as chemical intermediates, for example in the preparation of pharmaceutically acceptable salts by ion-exchange. Thus suitable salts of the compounds of the formula (II) include the lithium, sodium, potassium, calcium and magnesium salts and salts of nitrogenous bases. Particularly suitable salts of this invention include the sodium and potassium salts. Antibacterially active esters of the compounds of the formula (II) are believed to be those cleavable by in-vivo hydrolysis to the compounds of the formula (II) or their salt. Such esters may be identified by administration to a test animal such as a rat or a mouse by intravenous administration and thereafter examining the test animals body fluids for the presence of the compound of the formula (II) or its salt.

Suitable esters of this type include those of the part formulae (a) and (b):

$$-CO\text{-}O\text{-}CHA_1\text{-}O\text{-}CO\text{-}A_2 \qquad (a)$$

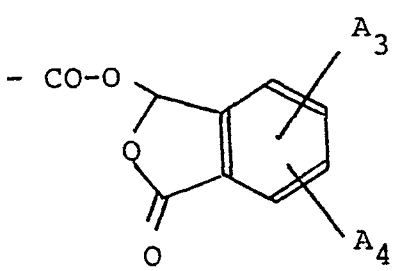

(b)

where $A_1$ is a hydrogen atom or a methyl group, $A_2$ is an alkyl or alkoxyl group of 1 - 4 carbon atoms or a phenyl group, $A_3$ is a hydrogen atom or a methyl or methoxyl group and $A_4$ is a hydrogen atom or a methyl or methoxyl group. Other esters of the compounds of the formula (II)

of interest are those cleavable by chemical methods as described hereinafter.

This invention also provides a process for the preparation of a compound of the formula (II) or a salt or cleavable ester thereof which comprises the ring closure with elimination of the elements of triphenylphosphineoxide from a cleavable ester of the compound of the formula (IV):

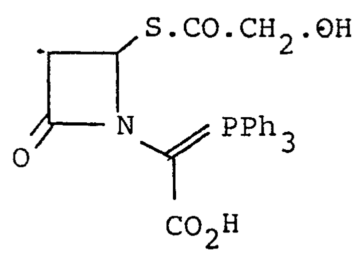

(IV)

in which the hydroxyl group is optionally protected and thereafter removing the optional protecting group and (a) cleaving the ester to yield a compound of the formula (III) or a salt thereof or (b) converting the hydroxyl group into a $OR_2$, $OCOR_3$, $OCOOR_4$. $OSO_2R_5$, $N_3$ or $NH_2$ group and if desired cleaving the ester of the resulting compound of the formula (II) to yield the compound of the formula (II) or a salt thereof.

The adaption of the preceding process to the preparation of the compound of the formula (III) or a salt or cleavable ester thereof is a preferred process aspect of this invention.

The ring closure reaction may be carried out on cleavable ester of a compound of the formula (IV) or on a corresponding compound in which the hydroxyl group is protected for example by silylation to form a tert-butyldiphenylsilyloxy group.

In general the ring closure is effected by heating in an inert solvent. Suitable solvents include

inert aromatic solvents such as toluene and temperatures of 60 - 120°, for example 100°, may be employed. The cyclised product may be obtained by evaporation of the solvent and thereafter purified chromatographically if desired.

If a tert-butyldiphenylsilyl group has been used as a protecting group this may be removed by reaction with anhydrous fluoride ion, for example as provided using a solution of tetrabutylammonium fluoride in tetrahydrofuran. The resulting ester of the compound of the formula (III) may be obtained from the reaction mixture by dilution with ethyl acetate, washing with water and evaporation of the organic layer.

The cleavable ester of the compound of the formula (IV) may be obtained by the following reaction sequence:

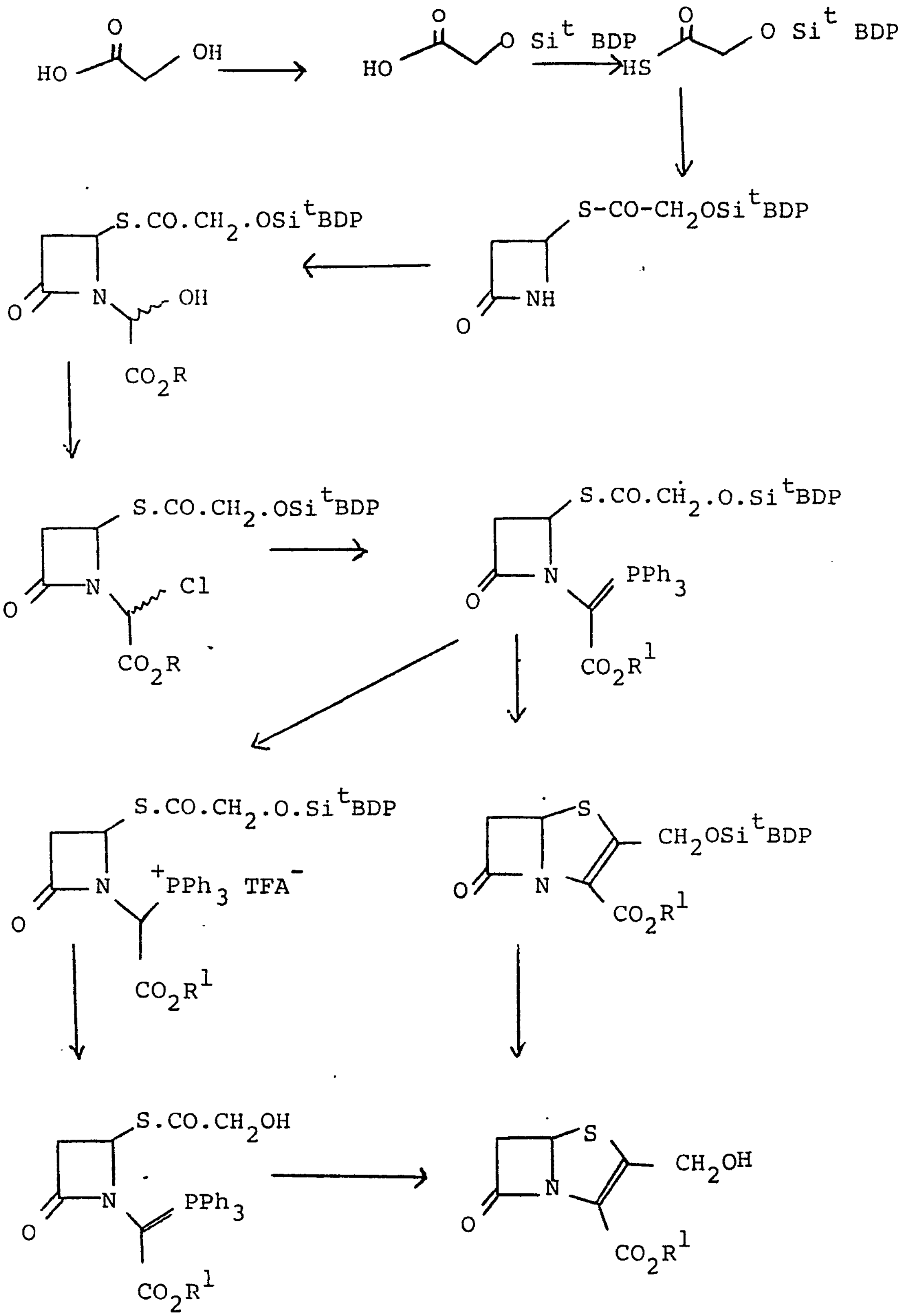

In the preceeding sequence $R^1$ is a group such that $CO_2R^1$ is a cleavable ester group.

Suitable cleavable ester groups include those removable by hydrogenolysis and those removable by hydrolysis. Esters conventionally removable by hydrogenolysis include substituted benzyl esters, such as the nitrobenzyl esters of which the p-nitrobenzyl ester is preferred. Such esters may be cleaved by hydrogenation, for example using palladium on charcoal as catalyst.

Particularly suitable esters for removable by hydrolysis include silyl esters such as the trimethylsilyl, tertbutyldiphenyl silyl and the like esters. The tertbutyldiphenylsilyl ester is particularly apt as it is readily cleavable by treatment with fluoride ion.

In a further aspect this invention provides a process for the preparation of a compound of the formula (III) or a salt thereof which comprises the hydrogenation of the p-nitrobenzyl ester of the compound of the formula (III) optionally in the presence of base.

The hydrogenation reaction may employ an approximately atmospheric pressure of hydrogen using a palladium catalyst such as palladium on charcoal, for example 5% palladium on charcoal. Conventional hydrogenation solvents may be used such as dioxane and water, for example a 4:1 mixture.

The initially produced compound of the formula (III) may be neutralised by reaction with a base which may be during the hydrogenation or introduced thereafter. Suitable bases include $LiCO_3$, LiOH, $Na_2CO_3$, $NaHCO_3$, $KHCO_2$, $Ca(OH)_2$, MgO or the like.

The desired salt may be obtained by diluting with a water immiscible solvent and extracting the salt into water. Evaporation of the aqueous solution yields the desired salt. Purification may be effected chromatographically, for example over Biogel P2.

BAD ORIGINAL

Further esters of the compound of the formula (III) may be prepared by esterifying a salt thereof, such as its sodium salt, with a reactive halide such as a bromide or chloride, for example phthalidyl bromide. Such reactions may be performed at ambient temperature in a conventional solvent such as dimethylformamide.

The general procedures leading to the preparation of cleavable esters of the compound of the formula (III) will be closely analogous to those set out in the Examples hereinafter for the preparation of the p-nitrobenzyl ester of the compound of the formula (III).

The cleavable esters of the compound of the formula (III) may be converted into the corresponding cleavable ester of a compound of the formula (II) wherein $R_1$ is a $OCOR_3$, $OCO_2R_4$ or $O.SO_2R_5$ group wherein $R_3$, $R_4$ and $R_5$ are as defined in relation to formula (II) by reaction with a corresponding compound of the formula (V), (VI) or (VII):

$$X.\ CO.R_3^1 \qquad (V)$$

$$X.\ CO_2R_4^1 \qquad (VI)$$

$$X.\ SO_2R_5 \qquad (VII)$$

wherein X is a good leaving group and $R_3^1$ and $R_4^1$ are groups $R_3$ and $R_4$ as defined in relation to formula (II) in which any reactive group is protected and $R_5$ is as defined in relation to formula (II) and thereafter removing any protecting group present in $R_3$ or $R_4^1$ Suitable values for X include Cl, Br, and, for (VI) and (VII), $OCOR_3^1$ and $OCO_2R_4^1$. Suitable protecting groups which may be employed in $R_3^1$ or $R_4^1$ include the benzyloxycarbonyl and p-nitrobenzyloxycarbonyl groups which may be removed by hydrogenation. In general the reaction of the

cleavable ester of the compound of the formula (III) will take place in the presence of an acid acceptor such as collidine, pyridine, triethylamine, potassium carbonate or the like. The reaction is normally effected at a non-extreme temperature, for example $0^{o}$-$50^{o}C$ in an organic solvent such as ethyl acetate or the like.

The cleavable esters of the compound of the formula (III) may be converted into the cleavable ester of a compound of the formula (II), wherein, $R_1$ is an $OR_2^{1}$ group where $R_2^{1}$ is a group $R_2^{1}$ as defined in relation to formula (II) wherein any reactive group is protected which process comprises reaction with an etherifying agent such as an alkylhalide in the presence of silver ions, a diazocompound in the presence of boron trifluoride, an epoxide, an acylated aziridine or the like and thereafter removing any protecting group present. Such etherifications may be effected in aprotic solvents such as dichloromethane or the like at a depressed or ambient temperature, for example $-70^{o}$ to $0^{o}C$. Suitable protecting groups include benzyloxycarbonyl and p-nitrobenzyloxycarbonyl groups which may be removed by hydrogenation.

The cleavable esters of the compound of the formula (II) wherein $R_1$ is a $N_3$ group may be prepared by the reaction of an ionic azide with a cleavable ester of a compound of formula (II) wherein $R_1$ is a $OSO_2R_5$ group. Suitable solvents for such reactions include polar organic solvents such as dimethylformamide. The reaction is normally carried out at a depressed temperature such as $-40^{o}$ to $0^{o}C$.

The compound of the formula (II) wherein $R_1$ is a $NH_2$ group may be prepared by hydrogenation of a benzyl or p-nitrobenzyl ester of the compound of the formula (II) wherein $R_1$ is a $N_3$ group.

Particularly apt cleavable esters of the compounds of the formula (II) for use in the substitution reactions include esters cleavable by hydrogenation such as the benzyl and p-nitrobenzyl esters. Such esters may be cleaved by hydrogenation in the presence of a noble metal catalyst such as palladium, for example palladium on charcoal. High, low or medium pressures of hydrogen may be employed but in general an atmospheric pressure of hydrogen has been favoured. The hydrogenation is conventionally carried out in a suitable solvent such as optionally aqueous ethanol, tetrahydrofuran, dioxan or the like.

Example 1

Benzyl O-t-butyldiphenylsilyl-glycollate (2)

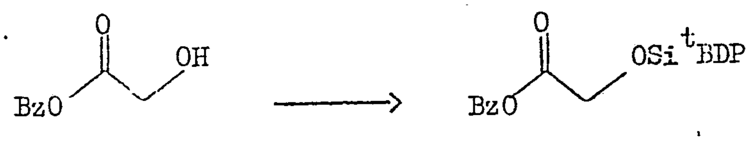

(1) (2)

To a stirred solution of benzyl glycollate (1) (4.15 g, 25 mmol) (J.C. Michaeu and A. Latles, Bull..Soc. Chim. Fr., 1970, 4018) and imidazole (3.74 g, 55 mmol) in dry DMF (200 mls) under argon was added a solution of t-butyldiphenylsilyl chloride (7.55 g, 27.5 mmol) in DMF (20 ml). After 1 hour the reaction mixture was poured into ethyl acetate (1,000 mls), washed well with water, dried ($Na_2SO_4$) and evaporated to give a yellow oil. Chromatography of the crude reaction mixture on silica gel eluting with ethyl acetate/petroleum* mixtures gave the ester (2) (7.4 g, 73%) as an oil. $\nu_{max.}$ ($CHCl_3$) 1750 $cm^{-1}$. δ ppm ($CDCl_3$) 1.10 (9H, s), 4.30 (2H, s); 5.15 (2H, s); 7.13 - 7.90 (15H, m).

* Denotes petroleum ether b.p. 60 - 80° throughout this Patent.
Also Bz denotes the benzyl group, $Si^tBDP$ denotes the t-butyldiphenylsilyl group, PNB denotes the p-nitrobenzyl group, and ∅ denotes the phenyl group.

Example 2

O-t-Butyldiphenylsilyl-glycollic acid (3) Method A

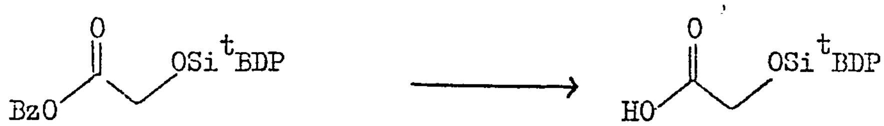

(2) (3)

The ester (2) (4.04 g, 10 mmol) in ethanol (100 ml) was hydrogenated over 10% palladium on charcoal catalyst (0.5 g) at N.T.P. until the theoretical amount of hydrogen had been consumed. The mixture was filtered through Kieselguhr and the residue washed with ethanol. The combined filtrates were evaporated to give the acid (3) (3 g). δ ppm ($CDCl_3$), 1.10 (9H, s), 4.30 (2H, s), 7.20 - 7.95 (10 H, m), 9.00 (1H, bs, exch. $D_2O$).

Method B

A solution of glycollic acid (2.28 g, 30 mmol) in DMF (300 ml) was sequentially treated with imidazole (5.10 g, 75 mmol) and t-butyldiphenylsilyl chloride (9.87 g, 36 mmol). After half an hour the reaction mixture was poured into ethyl acetate (1.5 litre), washed well with 5N HCl, dried ($Na_2SO_4$) and evaporated to give the acid (3) 8.40 g). The material was essentially the same as that prepared in Example 2, and was of sufficient purity for further synthetic work.

Example 3

O-t-Butyldiphenyl silyl-thioglycollic acid (4)

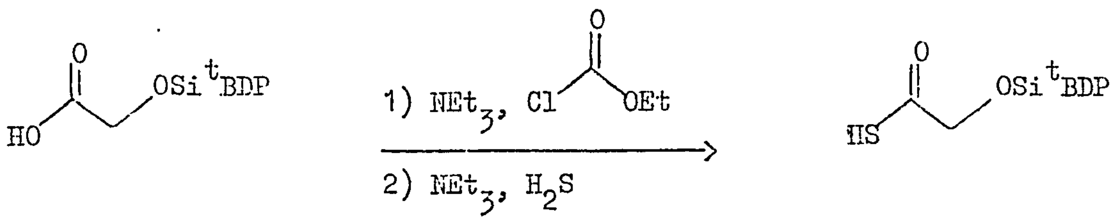


(3) DCM (4)

A solution of the acid (3) (2.22 g) and triethylamine (1 ml) in dry dichloromethane (50 ml) was stirred at room temperature for ten minutes. The solution was then cooled to -15° and ethyl chloroformate (0.7 ml) was added. After a further twenty minutes, more triethylamine (1 ml) was added and hydrogen sulphide was passed into the reaction mixture. After thirty minutes the mixture was warmed to room temperature, solvent was removed and toluene (50 ml) added. The mixture was filtered and the filtrate evaporated to give the triethylamine salt of the thioacid (4). This was redissolved in ether (50 ml), washed with 5N HCl, dried ($Na_2SO_4$) and evaporated to give the thioacid (4) (2.1 g) as an oil. $\nu_{max.}$ ($CHCl_3$) 2560, 1690 $cm^{-1}$. $\delta$ ppm ($CDCl_3$) 1.15 (9H, s) 4.20 (2H, s), 4.92 (1H, bs, exch. $D_2O$), 7.25 - 8.00 (10H, m).

Example 4



4-t-Butyldiphenylsilyloxyacetothio-azetidin-2-one (6)

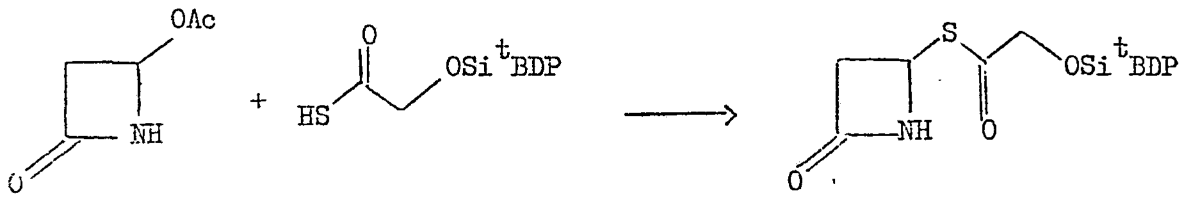

(5) (4) (6)

To a solution of sodium hydroxide (0.28 g) in water (30 ml) at 5° was added the thioacid (4) (2.1 g), followed after ten minutes by 4-acetoxy-azetidin-2-one (5) (0.9 g) in dichloromethane (30 ml). The two-phase mixture was vigorously stirred and warmed to room temperature over one hour. Dilute citric acid solution (10 ml) was added and the organic phase separated. The aqueous phase was further extracted with dichloromethane (3 x 15 ml). The combined organic phase was dried ($Na_2SO_4$), evaporated and chromatographed on silica gel eluting with ethyl acetate/petroleum mixtures gave the azetidinone (6) (1.15 g) as a colourless gum which slowly solidified, m.p. 57 - 9° (plates from ethyl acetate/petroleum); $\lambda_{max.}$ (EtOH) 222 nm (Em 17,500), $\nu_{max.}$ ($CHCl_3$) 3420, 1770, 1690 $cm^{-1}$, $\delta$ ppm ($CDCl_3$) 1.12 (9H, s), 3.00 (1H, ddd, J = 1, 2.5, 15 Hz, which collapses to 1H, dd, J = 2.5, 15 Hz on $D_2O$ exch.), 3.46 (1H, ddd, J = 1.5, 5, 15 Hz, collapses to 1H, dd, J = 5, 15 Hz on $D_2O$ exch.), 4.22 (2H, s), 5.16 (1H, dd, J = 2.5, 5 Hz), 6.40 (1H, bs, exch. $D_2O$), 7.24 - 7.80 (10H, m). (Found: C, 63.22; H, 6.39; N, 3.27; S, 7.99%. $C_{21}H_{25}NO_3SSi$ requires C, 63.12; H, 6.31; N, 3.51; S, 8.02%.

Example 5

4-t-Butyldiphenylsilyloxyacetothio-1-(1-hydroxy-1-benzyloxycarbonyl methyl)-azetidin-2-one (7)

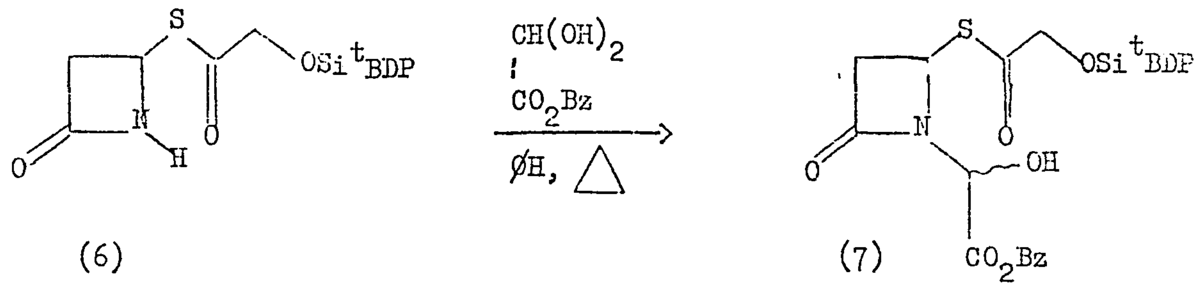


(6) (7)

A solution of the azetidinone (6) (1.1 g, 2.76 mmol) and benzyl glyoxylate (0.94 g, 5.94 mmol) in benzene (70 ml) was refluxed with provision for azotropic removal of water for eighteen hours. The reaction mixture was washed with water, dried ($Na_2SO_4$), evaporated and chromatographed on silica eluting with ethyl acetate/petroleum mixtures. This gave the hydroxy ester (7) (1.35 g) (87%) as a 1 : 1 mixture of epimers. $\nu_{max.}$ ($CHCl_3$) 3600 - 3200, 1760, 1690 $cm^{-1}$. δ ppm ($CDCl_3$) 1.11 (9H, s), 3.02 and 3.09 (1H, dd, J = 3, 15 Hz, trans-C3-H epimers), 3.40 and 3.47 (1H, dd, J = 5, 15 Hz, cis-C3-H epimers), 3.89 and 4.27 (1H, d, J = 8 Hz, exch. $D_2O$, -OH epimers), 4.22 (2H, s), 5.04 and 5.29 (centres of ABq, J = 12 Hz) and 5.27 (s), (2H, $-CO_2CH_2Ph$ epimers) 5.40 and 5.50 (1H, dd, J = 3, 5 Hz, C4-H epimers), 5.47 (1H, d, J = 8 Hz, collapses to 1H, s, on $D_2O$ exch.) 7.30 - 7.50 (15H, m).

Example 6



4-t-Butyldiphenylsiloxyacetothio-1-[1-chloro-1-benzyl oxycarbonyl methyl)-azetidin-2-one (8)

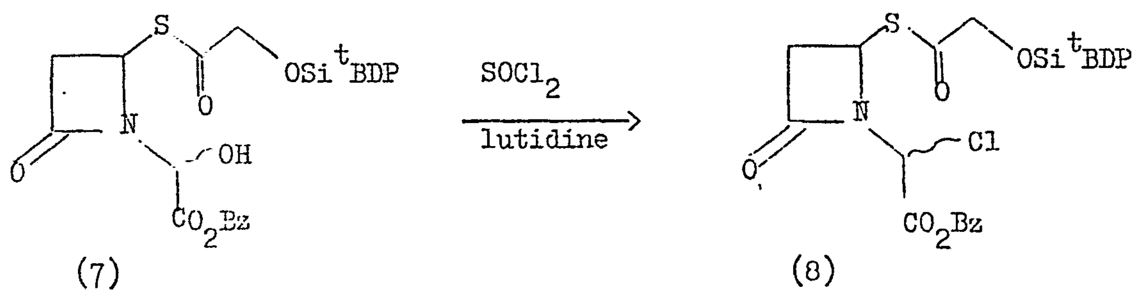


(7) (8)

A stirred solution of the hydroxy esters (7) (1.13 g, 2 mmol) in THF (50 ml) at -10° was sequentially treated with lutidine (0.35 ml, 3 mmol) and thionyl chloride (0.22 ml, 3 mmol). A white precipitate immediately formed. After twenty minutes the reaction mixture was filtered and the solid washed with THF (10 ml). The filtrate was evaporated, redissolved in toluene (50 ml) and filtered. The fitrate was evaporated to give a 1 : 1 mixture of the chloro epimers (8) (1.2 g) as a yellow oil. $\nu_{max.}$ ($CHCl_3$) 1780, 1700 $cm^{-1}$. δ ppm ($CDCl_3$) 1.11 (9H, s), 3.10 and 3.14 (1H, dd, J = 25, 16 Hz), 3.61 and 3.63 (1H, dd, J = 5, 15 Hz), 4.22 (2H, s), 5.10 and 5.24 (centres of ABq, J = 12 Hz) and 5.27 (s) (2H, in total), 5.61 and 5.64 (1H, dd, J = 2.5, 5 Hz) 6.02 and 6.07 (1H, s), 7.18 - 7.71 (15H, m).

Example 7

4-t-Butyldiphenylsilyoxyacetothio-1-[1-benzyloxycarbonyl-1-triphenyl phosphorylidene methyl]-azetidin-2-one (9)

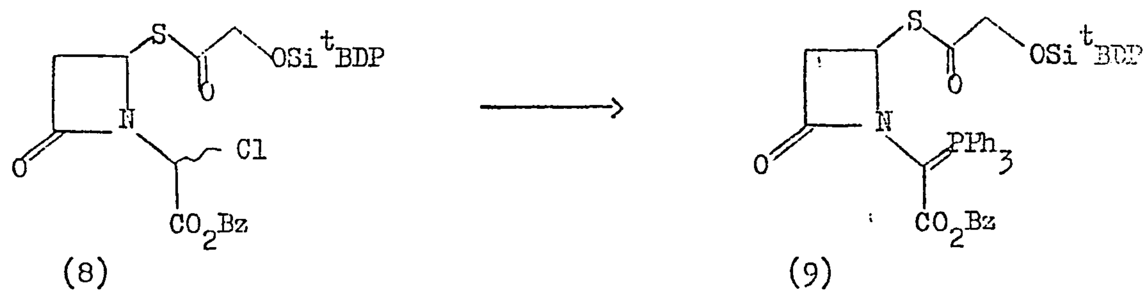

(8) (9)

A solution of chloro ester epimers (8) (1.16 g, 2 mmol), lutidine (0.28 ml, 2.4 mmol) and triphenyl phosphine (1.05g, 4 mmol) in dry dioxane (40 ml) was stirred under argon at 50° for eighteen hours. The reaction mixture was filtered and the solid washed with dioxane (10 ml). The filtrate was evaporated, dissolved in ethyl acetate (50 ml), washed with 2N HCl, brine, dried ($Na_2SO_4$) and evaporated. Chromatography on silica gel eluting with ethyl acetate/petroleum mixtures gave the phosphorane (9) (0.73 g, 45%) as a colourless oil which foamed. $\nu_{max.}$ ($CHCl_3$) 1740, 1690, 1680, 1610 $cm^{-1}$. (Found: C, 71.22; H, 5.54; N, 1.77; S, 3.82%. $C_{48}H_{46}NO_5PSSi$ requires C, 71.35; H, 5.74; N, 1.73; S, 3.97%).

Example 8

Benzyl 2-(t-Butyldiphenylsiloxymethyl) penem-3-carboxylate (10)

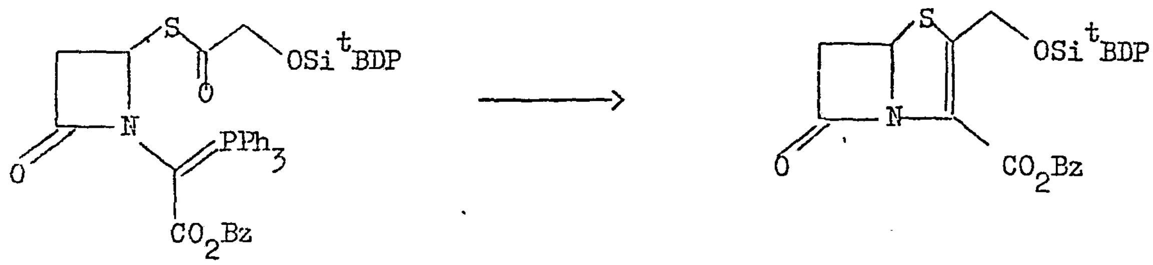

(9) (10)

A solution of the phosphorane (9) (528 mg, 0.65 mmol) was refluxed in dry toluene (300 ml) under argon for 2½ hours. The solvent was removed and the mixture chromatographed on silica gel eluting with ethyl acetate/petroleum mixtures to give the penem (10) (230 mg, 68%) as a gum that slowly solidified, m.p. 89 - 90°(needles from ethyl acetate/petroleum). $\lambda_{max.}$ (EtOH) 265 (Em 2,360), 323 (8,050) nm. $\nu_{max.}$ ($CHCl_3$) 1785, 1700, 1580 $cm^{-1}$. δ ppm ($CDCl_3$), 1.04 (9H, s), 3.44 (1H, dd, J = 2, 16 Hz), 3.78, 5.56 (1H, dd, J = 2, 4 Hz), 7.20 - 7.78 (15H, m). (Found: C, 67.83; H, 5.90; N, 2.87; S, 6.10%. $C_{30}H_{31}NO_4SSi$ requires, C, 68.02; H, 5.90; N, 2.64; S, 6.05%).

Example 9

Benzyl-2-Hydroxymethyl penem-3-carboxylate (11)

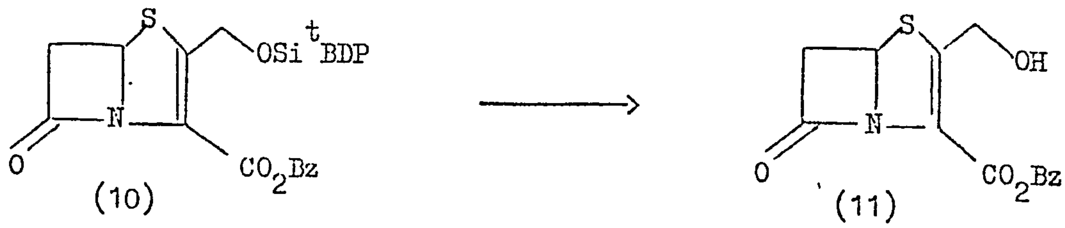

(10) (11)

A solution of the penem (10) (18 mg) and tetraethylammonium fluoride (containing 20 - 25% water) (20 mg) in THF (5 ml) was stirred at room temperature for two hours. Ethyl acetate (15 ml) was added and the mixture washed well with water, dried ($Na_2SO_4$) and evaporated. Chromatography on silica gel eluting with ethyl acetate/petroleum mixtures afforded the 2-hydroxymethyl penem benzyl ester (11). $\nu_{max.}$ ($CHCl_3$) 3600 - 3000, 1790, 1700, 1575 $cm^{-1}$.

Example 10



4-t-Butyldiphenylsiloxyacetothio-1-(1-p-nitrobenzyloxycarbonyl-1-hydroxymethyl)-azetidin-2-one (12)

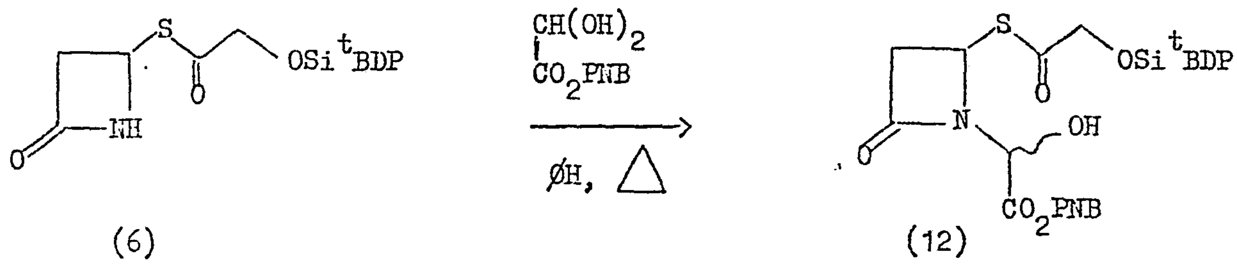


(6) (12)

A solution of the azetidinone (6) (4.00 g, 10 mmol) and p-nitrobenzyl glyoxylate (4.54 g, 20 mmol) in benzene (150 ml) was refluxed with provision for azeotropic removal of water for eighteen hours. The reaction mixture was washed with water, dried ($Na_2SO_4$) and evaporated. Chromatography on silica gel eluting with ethyl acetate/petroleum mixtures gave an epimeric mixture of the hydroxy-esters (12) (4.65 g, 76%) as a foam. $\nu_{max.}$ ($CHCl_3$) 3600 - 3200, 1775, 1695, 1520, 1350 $cm^{-1}$. δ ppm ($CDCl_3$) 1.18 (9H, s), 2.90 - 3.30 (1H, m), 3.30 - 3.68) (1H, m), 3.50 - 4.20 (1H, bs, exch. $D_2O$), 4.25 (2H, s), 5.20 - 5.65 (4H, m), 7.20 - 7.80 (12H, m), 8.05 - 8.33 (2H, m)

Example 11



4-t-Butyldiphenylsilyoxyacetothio-1-(1-p-nitrobenzyloxycarbonyl-1-chloromethyl)-azetidin-2-one (13)

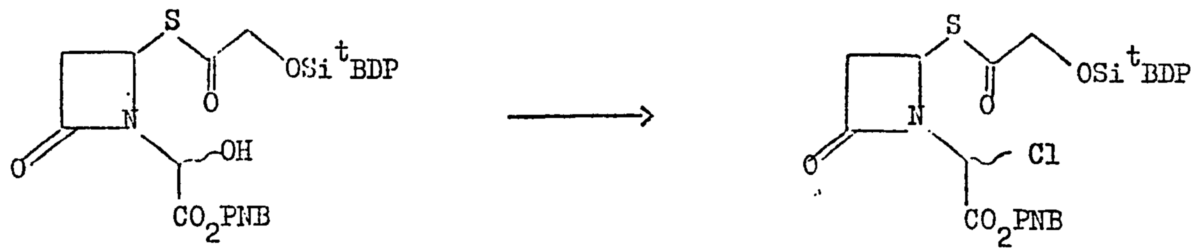

(12) (13)

A stirred solution of the hydroxy esters (12) (3.5 g, 5.76 mmol) in THF (200 ml) at -10° under argon was sequentially treated with lutidine (1.04 ml, 8.63 mmol) and thionyl chloride (0.63 ml, 8.63 mmol). A white precipitate immediately formed. After twenty minutes the reaction mixture was filtered and the solid washed with THF (50 ml). The filtrate was evaporated, redissolved in toluene (200 ml) and filtered. The filtrate was again evaporated to give the chloro esters (13) (3.6 g) as a yellow gum. $\nu_{max.}$ ($CHCl_3$) 1775, 1695, 1520, 1350 $cm^{-1}$. $\delta$ ppm ($CDCl_3$) (3 : 2 epimer ratio; a : b) 1.20 (9H, s), 3.11[a] and 3.16[b] (1H, dd, J = 4, 16 Hz); 3.60[b] and 3.64[a] (1H, dd, J = 6, 16 Fz), 4.22 (2H, s), 5.23[a] and 5.36[b] (2H, bs), 5.56 - 5.73 (1H, m), 6.06[a] and b.10[b] (1H, s), 7.33 - 7.64 (12H, m), 8.09 - 8.27 (2H, m).

Example 12



4-t-Butyldiphenylsilyoxyacetothio-1-(1-p-nitrobenzyloxycarbonyl-1-triphenylphosphorylidenemethyl)-azetidin-2-one (14)

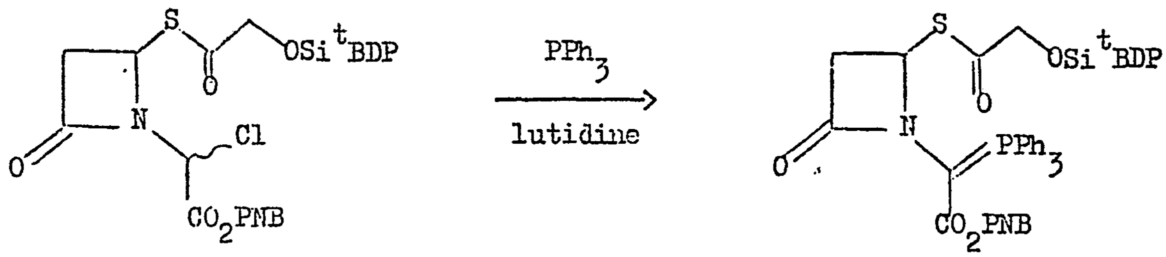


(13) (14)

A solution of the chloro ester epimers (13) (3.6 g, 5.75 mmol), lutidine (0.83 ml, 6.9 mmol) and triphenyl phosphine (3.02 g, 11.5 mmol) in dry dioxane (150 ml) were stirred under argon at 45° for eighteen hours. The reaction mixture was filtered and the solid washed with dioxane (50 ml). The filtrate was evaporated, dissolved in ethyl acetate (200 ml), washed with 2N HCl and brine. The dried organic phase was evaporated and chromatographed on silica gel eluting with ethyl acetate/petroleum mixtures to give the phosphorane (14) (3.5 g, 71%) as a yellow foam. $\nu_{max.}$ ($CHCl_3$) 1740, 1690 - 1680, 1615, 1515, 1345 $cm^{-1}$.

Example 13

4-t-Butyldiphenylsilyoxyacetothio-1-[1-p-nitrobenzyloxycarbonyl-1-triphenylphosphorylidenemethyl)-azetidin-2-one Trifluoracetic acid salt (15)

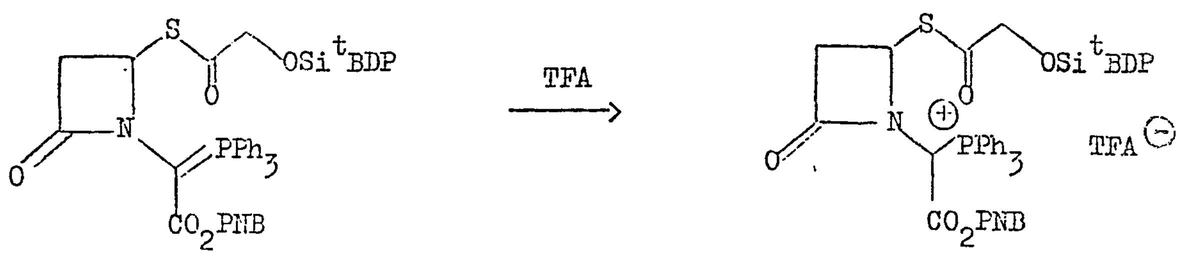


(14) (15)

To a stirred solution of the phosphorane (14) (3.6 g, 4.22 mmol) in ethyl acetate (250 ml) was added trifluoroacetic acid (17 mls, 4 eq.). After fifteen minutes, the solvent was removed, toluene (250 ml) was added, and the solvent re-evaporated. This process was repeated four times to give the phosphonium salt (15) (4.35 g) as a light yellow foam. $\nu_{max.}$ ($CHCl_3$) 1750, 1170 $cm^{-1}$.

Example 14

4-Hydroxyacetothio-1-(1-p-nitrobenzyoxycarbonyl-1-triphenylphosphorylidinemethyl)-azetidin-2-one (16)

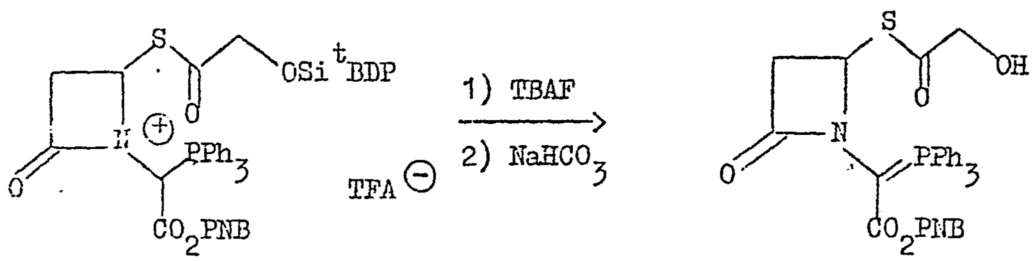


(15) (16)

A stirred solution of the phosphonium salt (15) (2.9 g, 3 mmol) in THF (150 ml) under argon was treated with a solution of tetrabutylammonium fluoride (9 mmol) in THF. After half an hour the mixture was evaporated, redissolved in ethyl acetate (200 ml) and washed with saturated sodium bicarbonate solution (3 x 100 ml), water (50 ml) and brine (100 ml). The dried organic phase was evaporated and chromatographed on silica gel eluting with ethyl acetate/petroleum mixtures to give the phosphorane (16) (1.3 g). $\nu_{max.}$ ($CHCl_3$) 3600 - 3000, 1750, 1685, 1620 $cm^{-1}$.

Example 15



p-Nitrobenzyl 2-Hydroxymethylpenem-3-carboxylate (17)  Method A

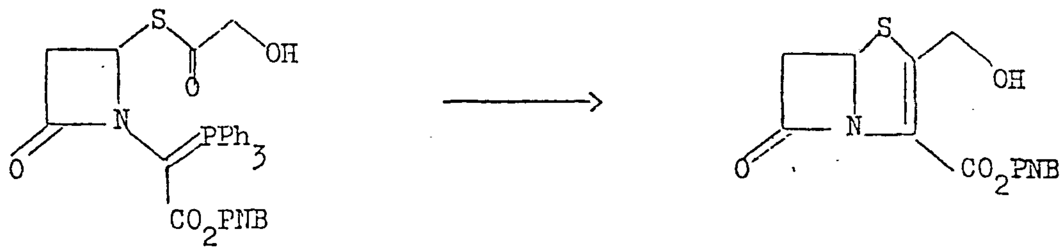

(16)  (17)

A solution of the phosphorane (16) (1.3 g) in purified toluene (700 ml) was stirred at 100° under argon. After one and three-quarter hours the solvent was removed and the residue chromatographed on silica gel eluting with ethyl acetate/petroleum mixtures to give the penem (17) (0.34 g) as a yellow solid. m.p. 137 - 9° (needles from ethyl acetate/petroleum, $\lambda_{max.}$ (EtOH), 265 (12,510), 322 (10,450). $\nu_{max.}$ ($CHCl_3$) 3600 - 3010, 1795, 1705, 1580 $cm^{-1}$. $\delta$ ppm ($CDCl_3$) 3.11 - 3.40 (1H, bs, exch. $D_2O$), 3.51 (1H, dd, J = 2, 16Hz), 3.84 (1H, dd, J = 4, 16Hz), 4.67 (2H, bs, collapses to s on $D_2O$ exch.), 5.22 and 5.47 (2H, centres of ABq, J = 14Hz) 5.67 (1H, dd, J = 2, 4Hz), 7.60 (2H, d, J = 8Hz), 8.20 (2H, d, J = 8Hz). (Found: C, 50.21; H, 3.43; N, 8.26; S, 9.52%. $C_{14}H_{12}N_2O_6S$ requires C, 50.00; H, 3.60; N, 8.33; S, 9.53%).

Example 16



p-Nitrobenzyl 2-(t-Butyldiphenylsilyloxymethyl) penem-3-carboxylate (18)

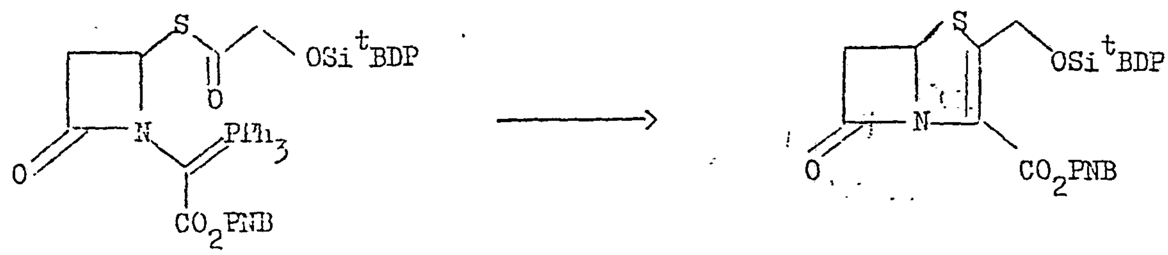

(14) (18)

A solution of the phosphorane (14) (400 mg) in dry toluene (300 ml) was refluxed under argon for two hours. The solvent was removed and the mixture chromatographed on silica gel:eluting with ethyl acetate/petroleum mixtures afforded the penem (18) (160 mgs, 60%) as an amorphous solid, m.p. 103 - 4° (needles from ether/petroleum). $\lambda_{max.}$ (EtOH) 268 (13,660), 324 (10,300), $\nu_{max.}$ 1785, 1700, 1575 $cm^{-1}$. $\delta$ ppm ($CDCl_3$) 1.07 (9H, 2) 3.50 (1H, dd, J = 2, 16Hz), 3.83 (1H, dd, J = 4, 16Hz), 4.86 (2H, s), 5.08 and 5.29 (2H, centres of ABq, J = 14Hz), 5.62 (1H, dd, J = 2 4Hz), 7.25 - 7.7 (12H, m) 8.11 (2H, d, J = 8Hz). (Found: C, 62.72; H, 5.35; N, 48.2; S, 5.36%. $C_{30}H_{30}N_2O_6SSi$ requires: C, 62.69; H, 5.26; N, 4.87; S, 5.58%

Example 17



p-Nitrobenzyl 2-Hydroxymethylpenem-3-carboxylate (17) Method B

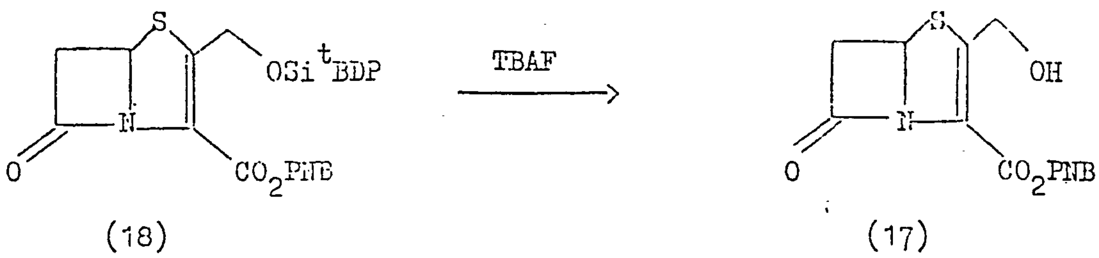


(18) (17)

A solution of penem (18) (100 mgs, 0.18 mmol) in THF (20 ml) under argon at -15° was treated with a solution of tetrabutylammonium fluoride (0.27 mmol) in THF. After half an hour the mixture was poured into ethyl acetate (50 ml) and washed with water (5 x 30 ml). The dried organic phase was evaporated and chromatographed on silica gel eluting with ethyl acetate/petroleum mixtures to give the penem (17) (14 mg). Material identical (i.r. and n.m.r. spectra) with that obtained *via* Method A.

Example 18



Sodium 2-Hydroxymethyl penem-3-carboxylate (19)

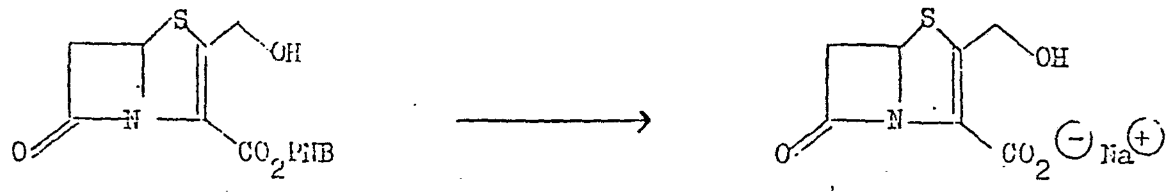

(17) (19)

The penem ester (17) (60 mg) was dissolved in a mixture of dioxane (6 ml) and water (1.5 ml) and hydrogenated over 5% palladium on charcoal catalyst (90 mg) for one hour. A further amount of catalyst (60 mg) was added and the hydrogenation continued for a further two hours. A 1% solution of sodium bicarbonate (1.35 ml) was added and the mixture filtered through Kieselguhr. The mixture was evaporated to low volume, water (15 ml) was added and the aqueous solution washed with a little ethyl acetate. Evaporation of the aqueous phase afforded the sodium salt (19) (30 mg) as an amorphous solid. $\lambda_{max.}$ (EtOH). 301 nm.

The minimum inhibitory concentrations (MIC) of this compound required to inhibit the growth of various bacteria are tabulated below:

| Organism | Agar[1] | Broth[2] |
|---|---|---|
| Citrobacter freundii E8 | 10 | |
| Enterobacter cloacae N1 | 50 | |
| Escherichia coli 0111 | 10 | 31 |
| Escherichia coli JT 39 | 10 | 62 |
| Klebsiella aerogenes A | 10 | 31 |
| Proteus mirabilis C977 | 25 | 62 |
| Proteus morganii 1580 | 25 | |
| Proteus rettgeri WM16 | 50 | |
| Proteus vulgaris W091 | 50 | |
| Pseudomonas aeruginosa A | 50 | 62 |
| Salmonella typhimurium CT10 | 10 | |
| Serratia marcescens US20 | 10 | |
| Shigella sonnei MB 11967 | 10 | |
| Bacillus subtilis A | 2.5 | |
| Staphylococcus aureus Oxford | 2.5 | 4.0 |
| Staphylococcus aureus Russell | 2.5 | 8.0 |
| Staphylococcus aureus 1517 | 25 | |
| Streptococcus faecalis I | 100 | |
| Streptococcus pneumoniae CN33 | 1.0 | |
| Streptococcus pyogenes CN10 | 1.0 | |
| E. coli ESS | 10 | |

1. DST agar + 10% horse blood
2. Microtitre using Nutrient broth

} inoculum 0.001 ml of a $10^{-2}$ dilution for G + ve bacteria or a $10^{-4}$ dilution for G - ve organisms.

Minimum Inhibitory Concentrations are expressed in microgrammes per ml.

Example 19

p-Nitrobenzyl 2-Acetoxymethyl penem-3-carboxylate (20)

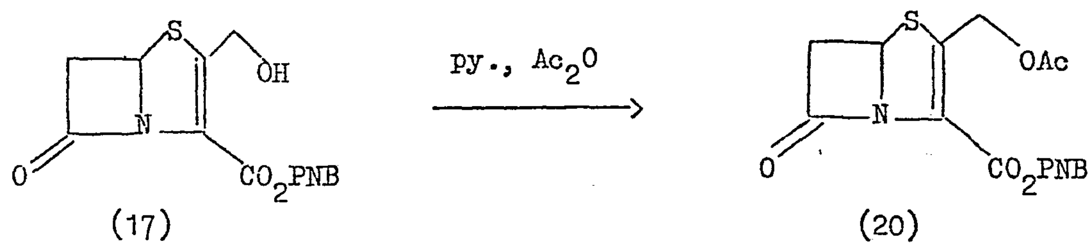


(17) (20)

A solution of the alcohol (17) (60 mg) in ethyl acetate (5 ml) was sequentially treated with pyridine (200 mg) and acetic anhydride (80 mg) and the mixture warmed to 40°. After three hours the mixture was washed with sodium bicarbonate solution (2 x 3 ml), citric acid solution (3 ml) and then water (3 ml). The dried organic phase was evaporated to give the acetate (20) (60 mg) as a light yellow foam. m.p. 125 - 7° (needles from ethyl acetate/petroleum) $\lambda_{max.}$ (EtOH), 265 (12,900), 323 (9,600) n.m. $\nu_{max.}$ ($CHCl_3$), 1800, 1745, 1715, 1590 $cm^{-1}$. δ ppm ($CDCl_3$) 2.15 (3H, s), 3.60 (1H, dd, J = 2, 15Hz), 3.95 (1H, dd, J = 4, 15Hz), 5.15 and 5.60 (2H, centres of ABq, J = 16Hz) 5.30 and 5.55 (2H, centres of ABq, J = 12Hz), 5.70 - 5.85 (1H, m), 7.70 (2H, d, J = 8Hz), 8.30 (2H, d, J = 8Hz). (Found: $M^+$ 378.0523. $C_{16}H_{14}N_2O_7S$ requires M 378.0519).

Example 20

Sodium 2-Acetoxymethyl penem-3-carboxylate (21)

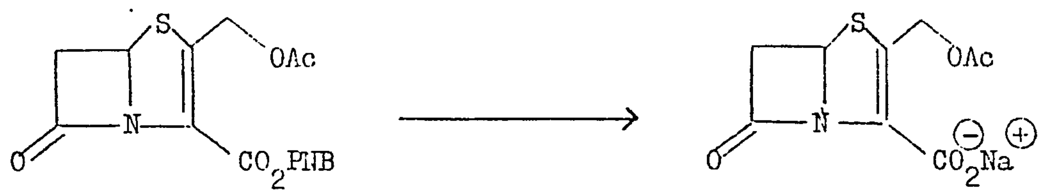

(20) (21)

The penem ester (20) (30 mg) was dissolved in a mixture of dioxane (4 ml) and water (1 ml) and hydrogenated over 5% palladium on charcoal catalyst (45 mg) for one hour. A further amount of catalyst (30 mg) was added and the hydrogenation was continued for a further one and a half hours. A 1% solution of sodium bicarbonate (0.6 ml) was added and the mixture filtered through Kieselguhr. The mixture was evaporated to low volume, water (10 ml) was added, and the aqueous solution washed with a little ethyl acetate. Evaporation of the aqueous phase afforded the sodium salt (21) (16 mg) as an amorphous solid, $\lambda_{max.}$ (EtOH) 298 n.m.

The minimum inhibitory concentrations (MIC) of this compound required to inhibit the growth of various bacteria are tabulated below:

| Organism | Agar[1] | Broth[2] |
|---|---|---|
| Citrobacter freundii E8 | 10 | |
| Enterobacter cloacae N1 | 100 | 62 |
| Escherichia coli 0111 | 25 | 8.0 |
| Escherichia coli JT 39 | 50 | 31 |
| Klebsiella aerogenes A | 10 | 8.0 |
| Proteus mirabilis C977 | 10 | 8.0 |
| Proteus morganii I580 | 10 | |
| Proteus rettgeri WM16 | 50 | |
| Proteus vulgaris W091 | 50 | |
| Pseudomonas aeruginosa A | >100 | 500 |
| Salmonella typhimurium CT10 | 25 | |
| Serratia marcescens US20 | 25 | |
| Shigella sonnei MB 11967 | 50 | |
| Bacillus subtilis A | 2.5 | |
| Staphylococcus aureus Oxford | 10 | |
| Staphylococcus aureus Russell | 10 | 8.0 |
| Staphylococcus aureus 1517 | 50 | 8.0 |
| Streptococcus faecalis I | 100 | |
| Streptococcus pneumoniae CN33 | 0.5 | |
| Streptococcus pyogenes CN10 | 2.5 | |
| E. coli ESS | 10 | |

1. DST agar + 10% horse blood
2. Microtitre using nutrient broth

} inoculum 0.001 ml of a $10^{-2}$ dilution for G + ve bacteria or a $10^{-4}$ dilution for G - ve organisms

Minimum Inhibitory Concentrations are expressed in microgrammes per ml.

0013067

p-Nitrobenzyl 2-(2-acetamidoethoxy)methylpenem-3-carboxylate (22)

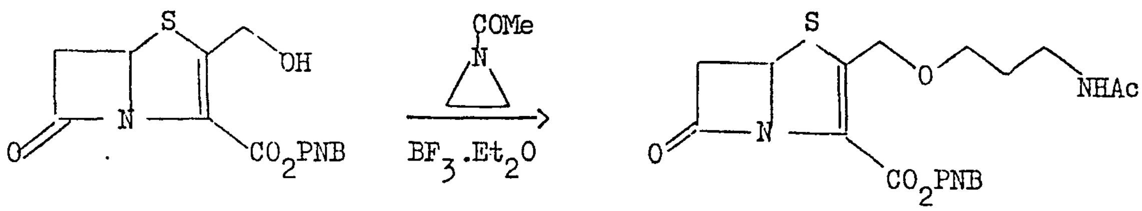


(17) (22)

A solution of the alcohol (17) (75 mg, 0.22 mmol) and N-acetylaziridine (57 mg, 0.6 mmol) in dry dichloromethane (10 ml) at -60° under argon was treated with freshly distilled boron trifluoride etherate (27 µl, 0.22 mmol). After fifteen minutes the mixture was slowly warmed to -10°. After one hour at -10°, the reaction mixture was cooled to -60° and sequentially treated with N-acetylaziridine (60 mg) and boron trifluoride etherate (25 µl) and the mixture warmed to -10° over forty-five minutes. The solution was then washed with sodium bicarbonate solution (3 x 10 ml), dried ($Na_2SO_4$) and evaporated. Chromatography on silica gel, eluting with ethyl acetate/petroleum mixtures afforded the ether (22) 65 mg, 70% as an amorphous solid, m.p. 133-4° (rosettes from ethyl acetate/petroleum). $\lambda_{max.}$ (EtOH) 261 (12,400), 319 (9,800) n.m. $\nu_{max.}$ ($CHCl_3$), 3450, 1790, 1705, 1660, 1580 $cm^{-1}$. $\delta$ ppm ($CDCl_3$) 2.02 (3H, s), 3.25 - 3.67 (5H, m), 3.85 (1H, dd, J = 6, 15Hz), 4.58 and 4.81 (2H, centres of ABq, J = 16Hz), 5.19 and 5.44 (2H, centres of ABq, J = 12Hz), 5.40 - 5.60 (1H, bs, exch., $D_2O$), 5.66 (1H, dd, J = 3, 6Hz), 7.57 (2H, d, J = 8Hz), 8.20 (2H, d, J = 8Hz). (Found: C, 51.29; H, 4.34; N, 9.77; S, 7.87%. $C_{18}H_{19}N_3O_7S$ requires: C, 51.30; H, 4.54; N, 9.97; S, 7.61%).

Example 22



Sodium 2-(2-acetamidoethoxy)methylpenem-3-carboxylate (23)

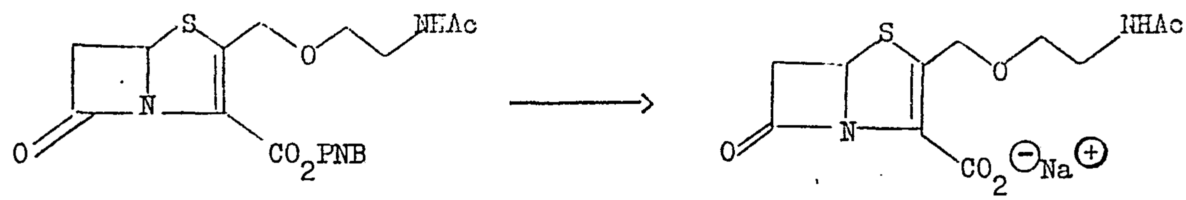

(22) (23)

The penem ester (22) (48 mg) was dissolved in a mixture of dioxane (5 ml) and water (1.25 ml) and hydrogenated over 5% palladium on charcoal catalyst (75 mg) for one hour. A further amount of catalyst (50 mg) was added and the hydrogenation was continued for a further one and a half hours. A 1% solution of sodium bicarbonate (0.86 ml) was added and the mixture filtered through Kieselguhr. The mixture was evaporated to low volume, water (15 ml) was added and the aqueous solution washed with a little ethyl acetate. Evaporation of the aqueous phase afforded the sodium salt (23) (25 mg) as an amorphous solid. $\lambda_{max.}$ (EtOH) 300 nm. The MIC of this compound required to inhibit the growth of various bacteria are tabulated below:-

| Organism | Agar[1] | Broth[2] |
|---|---|---|
| Citrobacter freundii E8 | 25 | |
| Enterobacter cloacae N1 | - | |
| Escherichia coli 0111 | 25 | 31 |
| Escherichia coli JT 39 | >100 | 250 |
| Klebsiella aerogenes A | 100 | 62 |
| Proteus mirabilis C977 | 25 | 31 |
| Proteus morganii I580 | >100 | |
| Proteus rettgeri WM16 | >100 | |
| Proteus vulgaris W091 | >100 | |
| Pseudomonas aeruginosa A | >100 | >500 |
| Salmonella typhimurium CT10 | 25 | . |
| Serratia marcescens US20 | 100 | |
| Shigella sonnei MB 11967 | - | |
| Bacillus subtilis A | 2.5 | |
| Staphylococcus aureus Oxford | 2.5 | 4.0 |
| Staphylococcus aureus Russell | 25 | 16 |
| Staphylococcus aureus 1517 | 50 | |
| Streptococcus faecalis I | >100 | |
| Streptococcus pneumoniae CN33 | 0.5 | |
| Streptococcus pyogenes CN10 | 1.0 | |
| E. coli ESS | 10 | |

1. DST agar + 10% horse blood
2. Mircotitre using Nutrient broth

} inoculum 0.001 ml of an undilute overmight broth culture.

MIC expressed in μg/ml

Example 23



p-Nitrobenzyl 2-methoxymethoxymethylpenem-3-carboxylate (24)

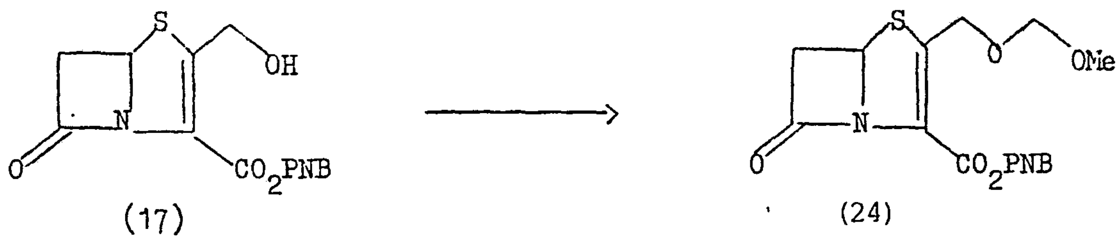

(17) (24)

A solution of the alcohol (17) (100 mg, 0.3 mmol), lutidine (320 mg, 3 mmol) and bromomethyl methyl ether (375 mg, 3 mmol)in dichloromethane (20 ml) was refluxed under argon for one hour. The reaction mixture was washed with sodium bicarbonate solution followed by citric acid solution and was then dried and evaporated. Chromatography on silica gel, eluting with ethyl acetate/petroleum mixtures afforded the ether (24) 65 mg, as a light yellow amorphous solid. m.p. 94 - 5° (light yellow needles from ethyl acetate/petroleum). $\lambda_{max.}$ (EtOH) 261 (12,400), 318 (10,000) nm. $\nu_{max.}$ ($CHCl_3$) 1795, 1705, 1580 $cm^{-1}$. $\delta$ ppm ($CDCl_3$) 3.36 (3H, s), 3.49 (1H, dd, J = 2, 16Hz), 3.83 (1H, dd, J = 4, 16Hz), 4.62 (2H, s), 4.64 and 4.88 (2H, centres of ABq, J = 16Hz); 5.20 and 5.44 (2H, centres of ABq, J = 13Hz), 5.65 (1H, dd, J = 2, 4Hz), 7.59 (2H, d, J = 8Hz), 8.20 (2H, d, J = 8Hz). (Found: C, 50.26; H, 4.14; N, 7.14; S, 8.60%. $C_{16}H_{16}N_2O_7S$ requires: C, 50.52; H, 4.24; N, 7.37; S, 8.43%).

Example 24

Sodium 2-methoxymethoxymethylpenem-3-carboxylate (25)

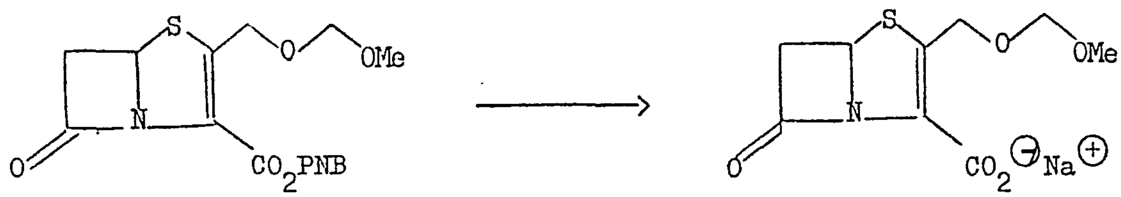

(24) (25)

The penem ester (24) (40 mg) was dissolved in a mixture of dioxane (5 ml) and water (1.25 ml) and hydrogeneated over 5% palladium on charcoal (60 mg) for fifty minutes. A further amount of catalyst (40 mg) was added and the hydrogenation was continued for a further one and three-quarter hours. A 1% solution of sodium bicarbonate (0.8 ml) was added and the misture filtered through Kieselguhr. The mixture was evaporated to low volume, water (15 ml) was added and the aqueous solution washed with a little ethyl acetate. The aqueous phase was evaporated to low volume and chromatographed on Biogel P2 200 - 400 mesh, eluting with water. The fractions were examined by u.v. and those of $\lambda_{max.}$ ($H_2O$), 305 and 255 nm were combined and evaporated to give the sodium salt (25) as an amorphous solid (19 mg). $\lambda_{max.}$ (EtOH) 258, 301 nm. $\nu_{max.}$ (KBr) 1770, 1580 $cm^{-1}$.

The MIC of this compound required to inhibit the growth of various bacteria are tabulated below:-

BAD ORIGINAL

| Organism | Agar[1] | Broth[2] |
|---|---|---|
| Citrobacter freundii E8 | 12.5 | |
| Enterobacter cloacae N1 | >50 | |
| Escherichia coli 0111 | 5.0 | 16 |
| Escherichia coli JT 39 | >50 | 125 |
| Klebsiella aerogenes A | 25 | 16 |
| Proteus mirabilis C977 | 12.5 | 16 |
| Proteus morganii I580 | >50 | |
| Proteus rettgeri WM16 | 25 | |
| Proteus vulgaris W091 | >50 | |
| Pseudomonas aeruginosa A | >50 | 500 |
| Salmonella typhimurium CT10 | 5.0 | |
| Serratia marcescens US20 | 25 | |
| Shigella sonnei MB 11967 | 5.0 | |
| Bacillus subtilis A | 1.2 | |
| Staphylococcus aureus Oxford | 2.5 | 2.0 |
| Staphylococcus aureus Russell | 5.0 | 8.0 |
| Staphylococcus aureus 1517 | >50 | |
| Streptococcus faecalis I | 50 | |
| Streptococcus pneumoniae CN33 | 0.2 | |
| Streptococcus pyogenes CN10 | 0.5 | |
| E. coli ESS | 2.5 | |

1. DST agar + 10% horse blood
2. Microtitre using Nutrient broth

} Inoculum 0.001 ml of an undilute overnight broth culture.

MIC values expressed in μg/ml

Example 25



p-Nitrobenzyl 2-mesyloxymethylpenem-3-carboxylate (26)

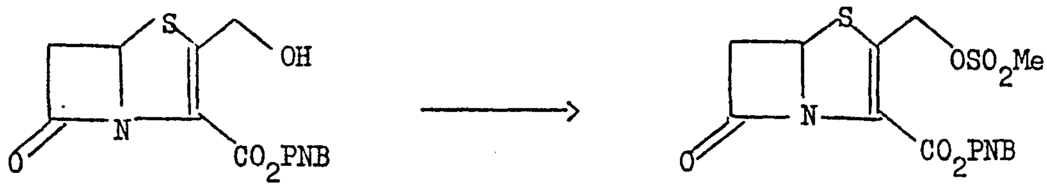

(17) (26)

A solution of the alcohol (17) (50 mg, 0.15 mmol) in dichloromethane (15 ml) at 0°C under argon was sequentially treated with triethylamine (45 mg, 0.45 mmol) and methanesulphonyl chloride (34 mg, 0.30 mmol). After ten minutes the reaction mixture was washed with sodium bicarbaonte solution (10 ml), citric acid solution (5 ml) and water. The dried organic phase was evaporated to give the crude mesylate (26) as a light yellow foam. $\nu_{max.}$ ($CHCl_3$) 1800, 1710, 1590, 1355, 1310, 1180 $cm^{-1}$. δ ppm ($CDCl_3$) 3.06 (3H, s), 3.55 (1H, dd, J = 2, 16Hz) 3.89 (1H, dd, J = 4, 16Hz), 5.29 and 5.60 (2H, centres of ABq, J = 15Hz), 5.34 and 5.45 (2H, centres of ABq, J = 13Hz), 5.75 (1H, dd, J = 2, 4Hz), 7.60 (2H, d, J = 8Hz), 8.20 (2H, d, J = 8Hz). The mesylate (26) was of sufficient purity for further synthetic work.

Example 26



p-Nitrobenzyl 2-azidomethylpenem-3-carboxylate (27)

Method A

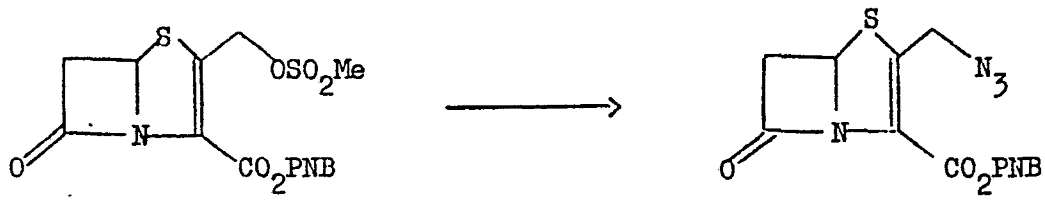

(26) (27)

A solution of sodium azide (8 mg) in DMF (10 ml) under argon at -40° was treated with a solution of the mesylate (26) (ex (17) (0.15 mmol)) in DMF (1 ml). After fifteen minutes the mixture was warmed to -10° and maintained at this temperature for one hour. The mixture was then poured into ethyl acetate (50 ml) and washed well with citric acid solution and brine. The dried organic phase was evaporated and chromatographed on silica gel, eluting with ethyl acetate/petroleum mixtures to give the azide (27) (20 mg) as a light yellow amorphous solid. m.p. 131 - 2° (needles from ethyl acetate/petroleum). $\lambda_{max.}$ (EtOH) 321 (6,500), 262 (9,300) nm. $\nu_{max.}$ ($CHCl_3$) 2130, 1800, 1715, 1585 $cm^{-1}$. $\delta$ ppm ($CDCl_3$) 3.56 (1H, dd, J = 2, 16Hz), 3.88 (1H, dd, J = 4, 16Hz), 4.42 and 4.78 (2H, centres of ABq, J = 15Hz), 5.23 and 5.47 (2H, centres of ABq, J = 13Hz), 5.72 (1H, dd, J = 2, 4Hz), 7.60 (2H, d, J = 8Hz), 8.23 (2H, d, J = 8Hz), (Found: $M^+$, 361.0486. $C_{14}H_{11}N_5O_5S$ requires M, 361.0481).

Method B

p-Nitrobenzyl-2-azidomethylpenem-3-carboxylate (27)

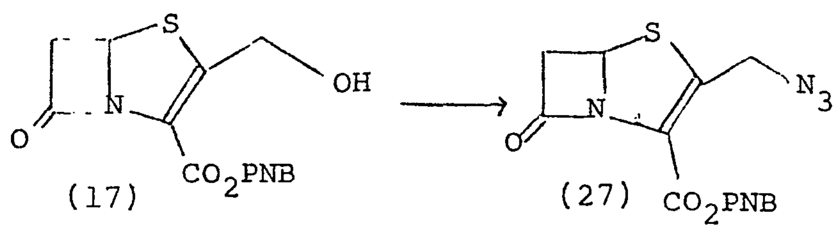

(17) (27)

A solution of the alcohol (17) (50 mg, 0.15 mmol) in dichloromethane (15 ml) at 0° under argon was sequentially treated with triethylamine (23 mg, 0.23 mmol) and methane sulphonyl chloride (26 mg, 0.23 mmol). After fifteen minutes the reaction mixture was cooled to -40° and treated with a solution of tetramethylguanidinium azide (36 mg, 0.23 mmol) in dichloromethane (1 ml). The reaction misture was then warmed to -10° and maintained at this temperature for 45 minutes. The mixture was then poured into dichloromethane (20 ml) and washed with dilute sodium bicarbonate solution (10 ml), dilute hydrochloric acid solution (10 ml) and brine (15 ml). The dried organic phase was evaporated and chromatographed on silica gel eluting with ethyl acetate/petroleum mixtures to give the azide (27) (16 mg).

Example 27

p-Nitrobenzyl 2-acetamidomethoxymethylpenem-3-carboxylate (28)

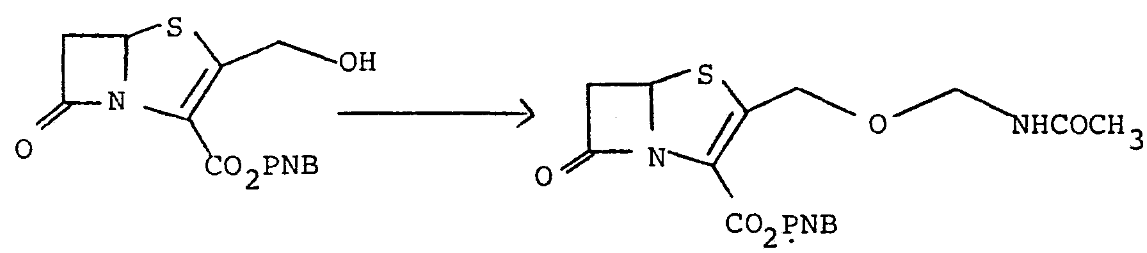

(17) (28)

A mixture of zinc acetate dihydrate (99 mg, 0.45 mmol) in benzene (30 ml) was refluxed with provision for azeotropic removal of water. After 15 minutes the refluxing mixture was sequentially treated with alcohol (17) (100 mg, 0.3 mmol) and acetoxymethylacetamide (132 mg, 0.96 mmol). After 2 hours a further amount of acetoxymethylacetamide (132 mg) was added to the reaction mixture. After a further 2 hours the reaction mixture was washed with sodium bicarbonate solution (10 ml) and brine (10 ml). The aqueous phase was extracted with dichloromethane (10 ml) and the combined organic phases were then dried and evaporated. Chromatography on silica gel eluting with ethyl acetate/petroleum mixtures afforded the ether (28), 26 mg, m.p. 152-3$^o$ (needles from ethyl acetate/petroleum), $\lambda_{max}$ (EtOH) 318 nm (Em 10,000), 262 (12,600), $\nu_{max}$ ($CHCl_3$) 3460, 1795, 1700, 1585 $cm^{-1}$; $\delta$ ppm ($CDCl_3$) 2.00 (3H, s), 3.49 (1H, dd, _J_ 2, 16Hz), 3.83 (1H, dd, _J_ 4, 16Hz), 4.62 and 4.86 (2H, centres of ABq, _J_ 15Hz), 4.70 (2H, d, _J_ 7Hz, collapses to bs on $D_2O$ exch.), 5.20 and 5.43 (2H, centres of ABq, _J_ 13 Hz), 5.65 (1H, dd, _J_ 2, 4Hz), 6.10-6.50 (1H, bs, exch), 7.60 (2H, d, _J_ 8Hz), 8.2 (2H, d, _J_ 8Hz). (Found: C, 49.84; H 4.17;

N, 10.24%; $M^+$ 407.0760. $C_{17}H_{17}N_3O_7S$ requires C, 50.12; H, 4.06; N, 10.31%; M 407.0784).

Example 28

Sodium 2-acetamidomethoxymethylpenem-3-carboxylate (29)

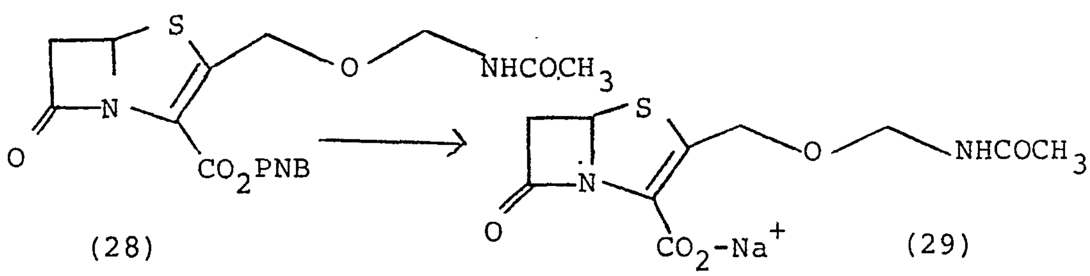

(28) (29)

The penem ester (28) (40 mg) was dissolved in a mixture of dioxane (5 ml) and water (1.25 ml) and hydrogenated over 5% palladium on charcoal (60 mg) for fifty minutes. A further amount of catalyst (40 mg) was added and the hydrogenation was continued for a further two and a half hours. A 1% solution of sodium bicarbonate (0.62 ml) was added and the mixture filtered through Kieselguhr. The mixture was evaporated to low volume, water (15 ml) was added and the aqueous solution washed with a little ethyl acetate. The aqueous phase was evaporated to low volume and chromatographed on Biogel P2 200-400 mesh, eluting with water. The appropriate fractions were combined and evaporated to give the sodium salt (29) as an amorphous solid (13 mg), $\lambda_{max}$ (EtOH) 301 nm (Em 4,100), 257 (2,700), $\nu_{max}$ (KBr) 1770, 1665, 1575 $cm^{-1}$.

The M.I.C. of this compound required to inhibit the growth of various bacteria are tabulated below:-

| Organism | Agar[1] | Broth[2] |
|---|---|---|
| Citrobacter freundii E8 | 5.0 | |
| Enterobacter cloacae N1 | >50 | |
| Escherichia coli 011 | 5.0 | 16 |
| Escherichia coli JT 39 | >50 | 125 |
| Klebsiella aerogenes A | 50 | 62 |
| Proteus mirabilis C977 | 12.5 | 31 |
| Proteus morganii I580 | >50 | |
| Proteus rettgeri WM16 | 50 | |
| Proteus vulgaris W091 | >50 | |
| Pseudomanas aeruginosa A | >50 | >500 |
| Salmonella typhimurium CT10 | 5.0 | |
| Serratia marcescens US20 | 25 | |
| Shigella sonnei MB 11967 | 5.0 | |
| Bacillus subtilis A | 1.2 | |
| Staphylococcus aureus Oxford | 2.5 | 8.0 |
| Staphylococcus aureus Russell | 12.5 | 16 |

Cont/d........

Cont/d.........

| Organism | Agar[1] | Broth[2] |
| --- | --- | --- |
| Staphylococcus aureus 1517 | 50 | |
| Streptococcus faecalis I | 50 | |
| Streptococcus pneumoniae CN33 | 0.2 | |
| Streptococcus pyogenes CN10 | 0.5 | |
| E. coli ESS | 2.5 | |

1. DST agar + 10% horse blood ) inoculum 0.001 ml of
2. Mircotitre using Nutrient broth ) an undilute overnight ) broth culture.

MIC values expressed in μg/ml

Example 29

p-Nitrobenzyl 2-(tetrahydropyran-2-yloxymethyl)penem-3-carboxylate (30)

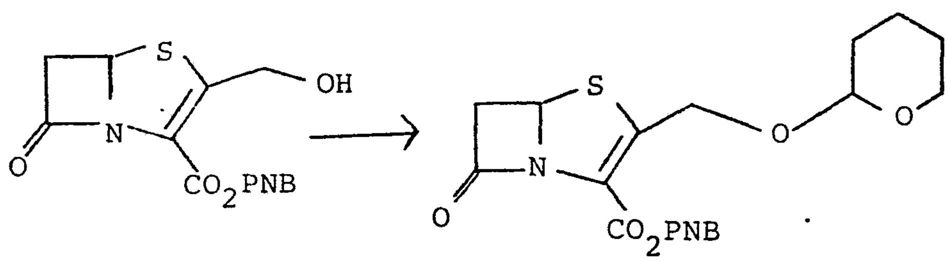

(17) (30)

A solution of the alcohol (17) (51 mg, 0.15 mmol), dihydropyran (26 mg, 0.30 mmol) and pyridinium p-toluenesulphonate (4 mg, 0.015 mmol) in dry dichloromethane (5 ml) was stirred at $10^{o}$ for 17 hours. Dichloromethane (15 ml) was added and the reaction mixture was washed with sodium bicarbonate solution (10 ml), brine (10 ml) and then dried and evaporated. Chromatography on silica gel eluting with ethyl acetate/petroleum mixtures afforded the ether (30), 50 mg, as a foam; $\lambda_{max}$ (EtOH) 319 nm (Em 6,300), 262 (8,000); $\nu_{max}$ ($CHCl_3$) 1798, 1715, 1585 $cm^{-1}$; $\delta$ ppm ($CDCl_3$) 1.30-1.95 (6H, m), 3.35-4.00 (4H, m), 4.55-4.82 (2H, m), 4.93 (1H, d, J 17 Hz), 5.19 and 5.43(2H, centres of ABq, J 14Hz), 5.64 (1H, dd, J 2, 4Hz), 7.58 (2H, d, J 8Hz), 8.21 (2H, d, J 8Hz). (Found: $M^+$ 420.0963. $C_{19}H_{20}N_2O_7S$ requires M 420.0989).

Example 30

Sodium 2-(tetrahydropyran-2-yloxylmethyl)penem-3-carboxylate (31)

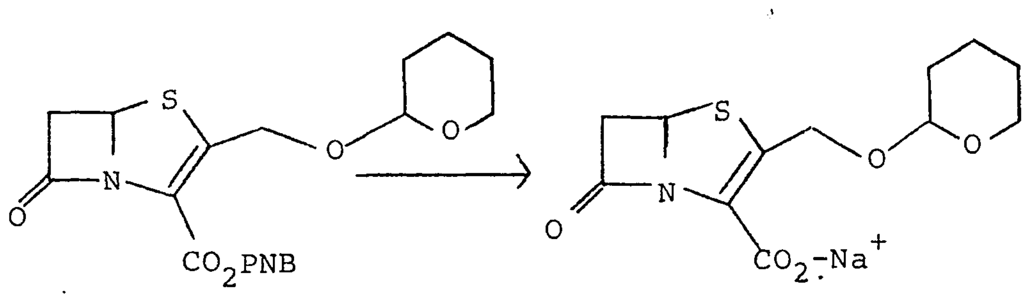

(30) (31)

The penem ester (30) (70 mg) was dissolved in a mixture of dioxane (7.5 ml) and water (2.25 ml) and hydrogenated over 5% palladium on charcoal (105 mg) for fifty minutes. A further amount of catalyst (70 mg) was added and the hydrogenation was continued for a further hour. A 1% solution of sodium bicarbonate (1.4 ml) was added and the mixture filtered through Kieselguhr. The mixture was evaporated to low volume and chromatographed on Biogel P2 200-400 mesh eluting with water. The appropriate fractions were combined and evaporated to afford the sodium salt (31) as an amorphous solid (45 mg). $\lambda_{max}$ ($H_2O$) 3Q3, 256 nm; $\nu_{max}$ (KBr) 1765, 1610, 1580 $cm^{-1}$.

Example 31

p-Nitrobenzyl 2-(1-methoxy-1-methylethoxy)methylpenem-3-carboxylate (32)

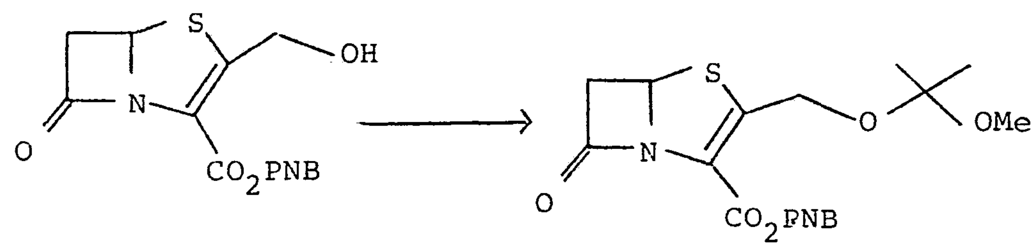

(17) (32)

A solution of the alcohol (17) (64 mg) and pyridinium p-toluenesulphonate (4 mg) in dry dichloromethane (5ml) was treated with excess of 2-methoxypropene (3 drops). After ten minutes, the reaction mixture was poured into dichloromethane (15 ml) and washed with sodium biçarbonate solution (10 ml) and brine (10 ml). The dried organic phase was evaporated and chromatographed on silica gel eluting with ethyl acetate/petroleum mixtures to give the ether (32), (62 mg), m.p. 105-6$^{o}$ (needles from ethyl acetate/petroleum/ether); $\lambda_{max}$ (EtOH) 320 nm (Em 9,800), 261 (11,700); $\nu_{max}$ ($CHCl_3$) 1795, 1710, 1580 $cm^{-1}$, $\delta$ ppm ($CDCl_3$) 1.34 (6H, s), 3.18 (3H, s), 3.46 (1H, dd, *J* 2, 16Hz), 3.81 (1H, dd, *J* 4, 16Hz), 4.55 and 4.77 (2H, centres of ABq, *J* 16Hz), 5.18 and 5.42 (2H, centres of ABq, *J* 14Hz), 5.62 (1H, dd, *J* 2, 4Hz), 7.60 (2H, d, *J* 8Hz), 8.20 (2H, d, *J* 8Hz). (Found : $M^+$ 408.0994. $C_{18}H_{20}N_2O_7S$ requires M 408.0991).

Example 32

p-Nitrobenzyl 2-(1-ethoxyethoxy)methylpenem-3-carboxylate (33)

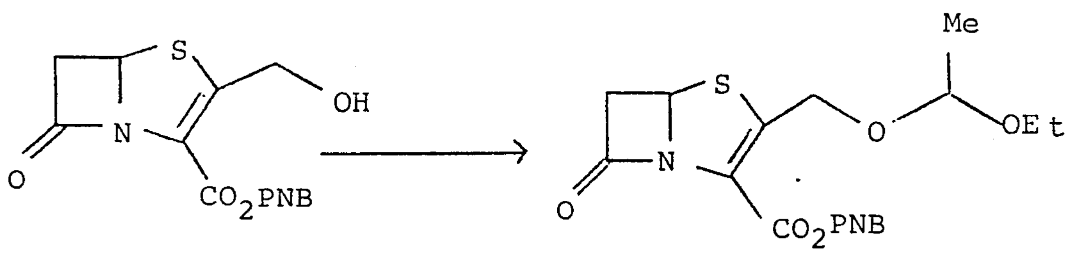

(17) (33)

A solution of the alcohol (17) (51 mg, 0.15 mmol) and pyridinium p-toluenesulphonate (6 mg, 0.025 mmol) in dry dichloromethane (8 ml) was treated with excess of ethyl vinyl ether (10 drops). After two hours the reaction mixture was poured into dichloromethane (10 ml) and brine (10 ml) and then dried and evaporated. Chromatography on silica gel eluting with ethyl acetate/petroleum mixtures afforded the ether (33), (50 mg), m.p. 92-98$^{o}$ (light yellow needles from ethyl acetate/petroleum); $\lambda_{max}$ (EtOH) 319 nm (Em 9,670), 262 (11,900); $\nu_{max}$ ($CHCl_3$) 1795, 1710, 1585 $cm^{-1}$; $\delta$ ppm ($CDCl_3$) 1.16 (3H, t, J 7Hz), 1.29 (3H, d, J 5.5 Hz), 3.28-3.70 (2H, m), 3.46 (1H, dd, J 2, 16.5Hz), 3.81 (1H, dd, J 4, 16.5Hz) 4.50-5.00 (3H, m), 5.20 and 5.45 (2H, centres of ABq, J 14Hz), 5.64 (1H, dd, J 2, 4Hz), 7.60 (2H, d, J 8Hz), 8.22 (2H, d, J 8Hz) (Found : $M^{+}$ 408.1012 $C_{18}H_{20}N_{2}O_{7}S$ requires M 408.0989).

Example 33

Sodium 2-(1-ethoxyethoxy)methylpenem-3-carboxylate (34)

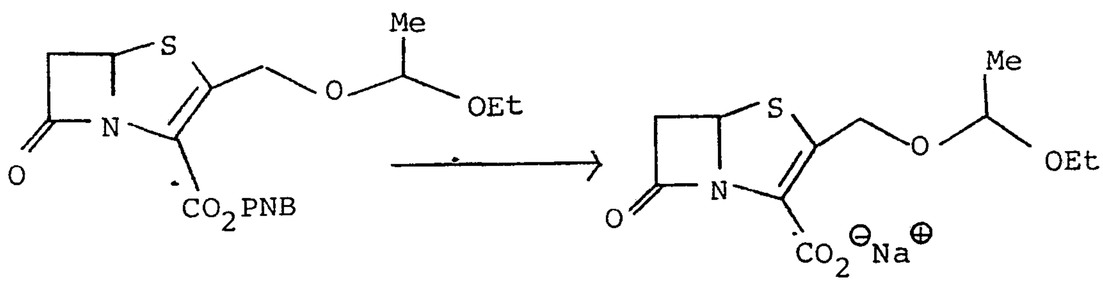

(33) (34)

The penem ester (33) (40 mg) was dissolved in a mixture of dioxane (5 ml) and water (1.5 ml) and hydrogenated over 5% palladium on charcoal (60 mg) for one hour. A further amount of catalyst (40 mg) was added and the hydrogenation was continued for a further one and a half hours. A 1% solution of sodium bicarbonate (0.84 ml) was added and the mixture filtered through Kieselguhr. The mixture was evaporated to low volume and chromatographed on Biogel P2 200-400 mesh eluting with water. The appropriate fractions were combined and evaporated to afford the sodium salt (34) as an amorphous solid (18 mg). $\lambda_{max}$ (EtOH) 299 nm (Em 4,200) 259 (3,500); $\nu_{max}$ (KBr) 1770, 1610, 1580 $cm^{-1}$.

The MIC of this compound required to inhibit the growth of various bacteria are tabulated below:-

| Organism | Agar[1] | Broth[2] |
|---|---|---|
| Staphylococcus aureus Oxford | 2.5 | |
| Staphylococcus aureus Russell | 10 | |
| Streptococcus pneumoniae CN33 | 1 | |
| Streptococcus pyogenes CN10 | 1 | |

1. DST agar + 10% horse blood ) inoculum 0.001 ml of
2. Mircotitre using Nutrient broth ) an undilute overnight ) broth culture.

MIC expressed in μg/ml

Example 34

p-Nitrobenzyl 2-N-methylcarbamoyloxymethylpenem-3-carboxylate (35)

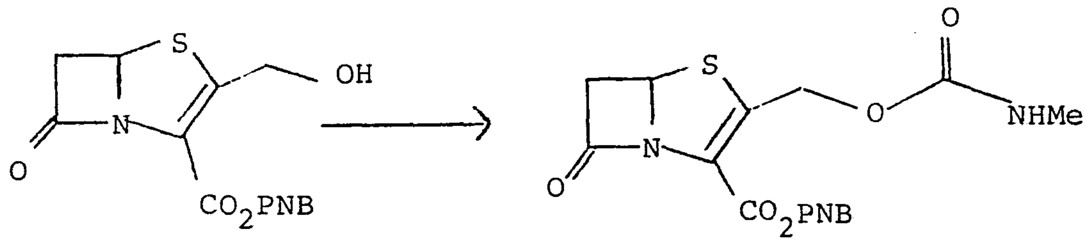

(17) (35)

A solution of the alcohol (17) (48 mg) and bis-tributyltin oxide (44 mg) in dry dichloromethane (5 ml) at $5^{o}$ was treated with a solution of methyl isocyanate (44 mg) in dichloromethane (1 ml). After 45 minutes the reaction mixture was poured into dichloromethane (10 ml) and washed with brine (10 ml). The dried organic phase was evaporated and then chromatographed on silica gel eluting with ethyl acetate/petroleum mixtures to give the carbamate (35), 46 mg, as an off-white amorphous solid, mp 126-$7^{o}$ (needles from ethyl acetate/petroleum), $\lambda_{max}$ (EtOH) 319 nm (Em 9,100), 262 (N, 800); $\nu_{max}$ ($CHCl_3$), 3460, 1795, 1720, 1585 $cm^{-1}$; $\delta$ ppm ($CDCl_3$), 2.78 (3H, d, *J* 5Hz), 3.49 (1H, dd, *J* 2, 16 Hz), 3.83 (1H, dd, *J* 4, 16Hz), 4.74 (1H, bs), 5.07 and 5.49 (2H, centres of ABq, *J* 16Hz), 5.22 and 5.46 (2H, centres of ABq, *J* 14Hz), 5.66 (1H, dd, *J* 2, 4Hz), 7.60 (2H, d, *J* 8Hz), 8.21 (2H, d, *J* 8Hz) (Found: $M^+$ - CO 365.0707 $C_{15}H_{15}N_3O_6S$ requires M 365.0680).

Example 35

Sodium 2-N-methylcarbamoyloxymethylpenem-3-carboxylate (36)

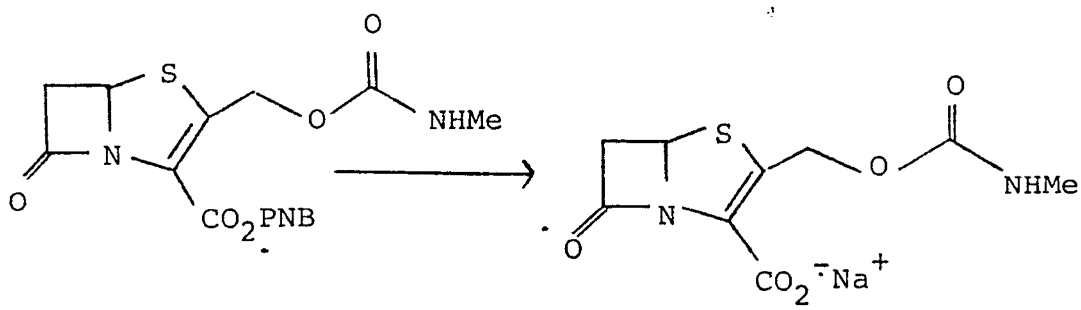

(35) (36)

The penem ester (35) (30 mg) was dissolved in a mixture of dioxane (3.6 ml) and water (0.9 ml) and hydrogenated over 5% palladium on charcoal (45 mg) for one hour. A further amount of catalyst (30 mg) was added and the hydrogenation continued for a further thirty minutes. A 1% solution of sodium bicarbonate (0.62 ml) was added and the mixture filtered through Kieselguhr. The mixture was evaporated to low volume and chromatographed on Biogel P2 200-400 mesh eluting with water. The appropriate fractions were combined and evaporated to afford the sodium salt (36) as an amorphous solid (13 mg). $\lambda_{max}$ (EtOH) 299 nm (Em 3,300), 258 (2,800); $\nu_{max}$ (KBr), 1765, 1710, 1600 $cm^{-1}$.

The MIC of this compound required to inhibit the growth of various bacteria are tabulated below:-

| Organism | Agar[1] | Broth[2] |
|---|---|---|
| Bacillus subtilis A | 2.5 | |
| Staphylococcus aureus Oxford | 12.5 | 8.0 |
| Staphylococcus aureus Russell | 12.5 | 16 |
| Streptococcus pyogenes CN10 | 25 | |

1. DST agar + 10% horse blood ) inoculum 0.001 ml of
2. Mircotitre using Nutrient broth ) an undilute overnight ) broth culture.

MIC expressed in μg/ml

Example 36

2-Aminomethylpenem-3-carboxylic acid (37)

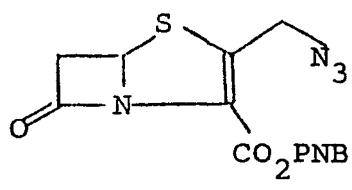

(27)

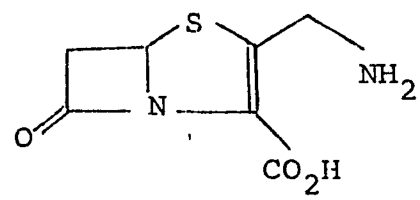

(37)

The penem ester (27) (16 mg) was dissolved in a mixture of dioxane (2.5 ml ) and water (0.75 ml) and hydrogenated over 5% palladium on charcoal (30 mg) for fifty minutes. A further amount of catalyst (20 mg) was added and the hydrogenation was continued for a further thirty minutes. The mixture was filtered through Kieselguhr and the filter pad washed well with 1:1 dioxane/water. The filtrate was evaporated, water was added and the solution chromatographed on XAD-2 resin eluting with water. The appropriate fractions were combined to give the acid (37), $\lambda$ max ($H_2O$) 309, 258 nm.

The M.I.C. of this compound required to inhibit the growth of various bacteria are tabulated below:-

| Organism | Agar[1] | Broth[2] |
|---|---|---|
| Citrobacter freundii E8 | 12.5 | 0.5 |
| Enterobacter cloacae N1 | 50 | 16 |
| Escherichia coli 011 | 25 | 8 |
| Escherichia coli JT 39 | 50 | 16 |
| Klebsiella aerogenes A | 50 | 8 |
| Proteus mirabilis C977 | 25 | 8 |
| Proteus morganii I580 | >50 | 16 |
| Proteus rettgeri WM16 | >50 | |
| Proteus vulgaris W091 | >50 | |
| Pseudomanas aeruginosa A | 12.5 | 1 |
| Salmonella typhimurium CT10 | 12.5 | |
| Serratia marcescens US20 | 25 | 16 |
| Shigella sonnei MB 11967 | 12.5 | 4 |
| Bacillus subtilis A | 2.5 | |
| Staphylococcus aureus Oxford | 5 | 2 |
| Staphylococcus aureus Russell | 5 | 4 |

Cont/d........

Cont/d.........

| Organism | Agar[1] | Broth[2] |
|---|---|---|
| Staphylococcus aureus 1517 | >50 | |
| Streptococcus faecalis I | >50 | 62 |
| Streptococcus pneumoniae CN33 | 0.5 | |
| Streptococcus pyogenes CN10 | 1.25 | 1 |
| E. coli ESS | 5 | 4 |

1. DST agar + 10% horse blood ) inoculum 0.001 ml of
2. Mircotitre using Nutrient broth ) an undilute overnight ) broth culture.

WHAT WE CLAIM IS:

1. A compound of the formula (II):

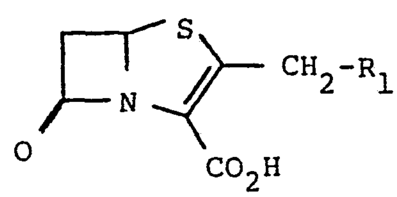

(II)

or a salt or cleavable ester thereof wherein $R_1$ is a OH, $OR_2$, $OCOR_3$, $O.CO.OR_4$, $O.SO_2R_5$, $N_3$ or $NH_2$ group wherein $R_2$ is a lower alkyl group, a substituted lower alkyl group or an aralkyl group; $R_3$ is a lower alkyl group, a substituted lower alkyl group, an aryl group or an aralkyl group; $R_4$ is a lower alkyl group, a substituted lower alkyl group, an aryl group or an aralkyl group; and $R_5$ is a lower alkyl group, or a tolyl group.

2. A compound as claimed in claim 1 wherein $R_1$ is OH.

3. A compound as claimed in claim 1 wherein $R_1$ is $OR_2$ where $R_2$ is a methyl, ethyl, benzyl, 2-hydroxyethyl, 2-aminoethyl, 2-acetoxyethyl, 2-methoxyethyl, acetoxymethyl, methoxymethyl or methane sulphonyl group.

4. A compound as claimed in claim 1 wherein $R_1$ is a $N_3$ group.

5. A compound as claimed in claim 1 wherein $R_1$ is a $NH_2$ group.

6. A compound of the formula (II) as claimed in any of claims 1 to 5 in the form of a pharmaceutically acceptable salt.

7. A compound of the formula (II) as claimed in any of claims 1 to 5 in the form of a cleavable ester wherein the ester moiety is of the part formulae (a) or (b):

$$- CO-O-CHA_1-O-CO-A_2 \qquad (a)$$

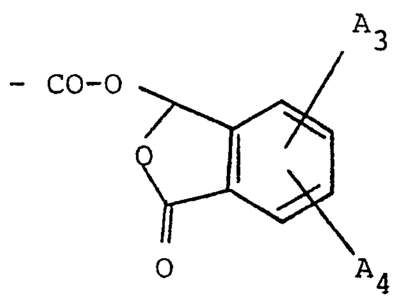

(b)

where $A_1$ is a hydrogen atom or a methyl group, $A_2$ is an alkyl or alkoxyl group of 1 - 4 carbon atoms or a phenyl group, $A_3$ is a hydrogen atom or a methyl or methoxyl group and $A_4$ is a hydrogen atom or a methyl or methoxyl group.

8. A compound of the formula (II) as claimed in any of claims 1 to 5 in the form of a benzyl or p-nitrobenzyl ester.

9. A pharmaceutical composition which comprises a compound as claimed in any of claims 1 to 7 and a pharmaceutically acceptable carrier therefor.

10. A process for the preparation of a compound of the formula (II) or a salt or cleavable ester thereof as claimed in any of claims 1 to 9 which comprises the ring closure with elimination of the elements of triphenylphosphineoxide from a cleavable ester of the compound of the formula (IV):

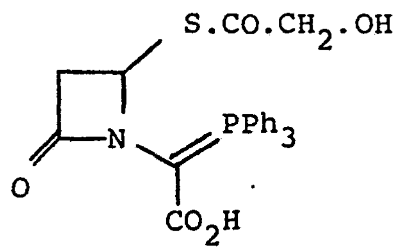

(IV) .

in which the hydroxyl group is optionally protected and thereafter removing the optional protecting group and (a) cleaving the ester to yield a compound of the formula (III) or a salt thereof or (b) converting the hydroxyl group into a $OR_2$, $OCOR_3$, $OCOOR_4$. $OSO_2R_5$, $N_3$ or $NH_2$ group and if desired cleaving the ester of the resulting compound of the formula (II) to yield the compound of the formula (II) or a salt thereof.

European Patent Office

# EUROPEAN SEARCH REPORT

0013067
Application number

EP 79 30 2384

## DOCUMENTS CONSIDERED TO BE RELEVANT

| Category | Citation of document with indication, where appropriate, of relevant passages | Relevant to claim |
|---|---|---|
| X | DE - A - 2 819 655 (CIBA-GEIGY)<br>* Claims 1-4, 11,24-26 * | 1,6-10 |
| P | EP - A - 0 000 636 (GLAXO)<br>* Claims 1-7 * | 1,3,<br>6-10 |
| P | EP - A - 0 003 415 (GLAXO)<br>* Claims 1-14 * | 1,4-10 |
| P | EP - A - 0 002 210 (MERCK)<br>* Claims 1-5 * | 1,6-10 |

CLASSIFICATION OF THE APPLICATION (Int. Cl.)

C 07 D 499/00
A 61 K 31/43//
C 07 D 205/08
C 07 F 7/18
9/65

TECHNICAL FIELDS SEARCHED (Int.Cl.)

C 07 D 499/00
A 61 K 31/43

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 04-03-1980 | CHOULY |

EPO Form 1503.1 06.78